(19) **Europäisches Patentamt / European Patent Office / Office européen des brevets**

(11) **EP 3 443 087 B1**

(12) **EUROPEAN PATENT SPECIFICATION**

(45) Date of publication and mention of the grant of the patent:
**18.09.2024 Bulletin 2024/38**

(21) Application number: **17783053.6**

(22) Date of filing: **12.04.2017**

(51) International Patent Classification (IPC):
**C12N 15/10** (2006.01)  **C12N 15/09** (2006.01)
**C07K 14/725** (2006.01)  **C07K 16/00** (2006.01)
**C12Q 1/68** (2018.01)  **C12Q 1/6806** (2018.01)

(52) Cooperative Patent Classification (CPC):
(C-Sets available)
**C07K 16/00; C07K 16/005; C12Q 1/6806;**
**C12Q 1/686;** C07K 2317/10; C07K 2317/622;
C07K 2319/00  (Cont.)

(86) International application number:
**PCT/US2017/027199**

(87) International publication number:
**WO 2017/180738 (19.10.2017 Gazette 2017/42)**

(54) **IMMUNE REPERTOIRE MINING**

IMMUNREPERTOIRE-GEWINNUNG

EXPLORATION DU RÉPERTOIRE IMMUN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **12.04.2016  US 201662321278 P**

(43) Date of publication of application:
**20.02.2019 Bulletin 2019/08**

(73) Proprietor: **Medimmune, LLC**
**Gaithersburg, Maryland 20878 (US)**

(72) Inventors:
• **CHOWDHURY, Partha, S.**
**Gaithersburg, MD 20878 (US)**
• **RAJAN, Saravanan**
**Gaithersburg, MD 20878 (US)**
• **KIERNY, Michael, Robert**
**Gaithersburg, MD 20878 (US)**

(74) Representative: **AstraZeneca Intellectual Property**
**Eastbrook House**
**Shaftesbury Road**
**Cambridge CB2 8BF (GB)**

(56) References cited:
WO-A1-2014/182197    WO-A2-2014/004124
US-A1- 2007 141 048    US-A1- 2010 285 975

• **LEVIN MATTIAS ET AL: "Persistence and evolution of allergen-specific IgE repertoires during subcutaneous specific immunotherapy", JOURNAL OF ALLERGY AND CLINICAL IMMUNOLOGY, ELSEVIER, AMSTERDAM, NL, vol. 137, no. 5, 11 November 2015 (2015-11-11), pages 1535 - 1544, XP029531146, ISSN: 0091-6749, DOI: 10.1016/J.JACI.2015.09.027**
• **MARIA A. TURCHANINOVA ET AL: "Pairing of T-cell receptor chains via emulsion PCR : New technology", EUROPEAN JOURNAL OF IMMUNOLOGY, vol. 43, no. 9, 26 June 2013 (2013-06-26), Weinheim, pages 2507 - 2515, XP055392257, ISSN: 0014-2980, DOI: 10.1002/eji.201343453**

**(Cont. next page)**

- BRANDON J DEKOSKY ET AL: "High-throughput sequencing of the paired human immunoglobulin heavy and light chain repertoire", NATURE BIOTECHNOLOGY, vol. 31, no. 2, 1 January 2013 (2013-01-01), pages 166 - 169, XP055054081, ISSN: 1087-0156, DOI: 10.1038/nbt.2492
- FERNANDA FESTA ET AL: "High-throughput cloning and expression library creation for functional proteomics", PROTEOMICS, vol. 13, no. 9, 5 April 2013 (2013-04-05), DE, pages 1381 - 1399, XP055716898, ISSN: 1615-9853, DOI: 10.1002/pmic.201200456
- ZHU QINGHE ET AL: "Expression and Purification of the scFv from Hybridoma Cells Secreting a Monoclonal Antibody Against S Protein of PEDV", MONOCLONAL ANTIBODIES IN IMMUNODIAGNOSIS AND IMMUNOTHERAPY, vol. 32, no. 1, 1 February 2013 (2013-02-01), US, pages 41 - 46, XP055909825, ISSN: 2167-9436, DOI: 10.1089/mab.2012.0089
- PABLO H. SOTELO ET AL: "An efficient method for variable region assembly in the construction of scFv phage display libraries using independent strand amplification", MABS, vol. 4, no. 4, 1 July 2012 (2012-07-01), US, pages 542 - 550, XP055622734, ISSN: 1942-0862, DOI: 10.4161/mabs.20653
- NAKANO M ET AL: "single-molecule PCR using water-in-oil emulsion", JOURNAL OF BIOTECHNOLOGY, ELSEVIER, AMSTERDAM NL, vol. 102, 1 January 2003 (2003-01-01), pages 117 - 124, XP002326273, ISSN: 0168-1656, DOI: 10.1016/S0168-1656(03)00023-3
- XIAO ET AL.: "A Novel Dual Expression Platform for High Throughput Functional Screening of Phage Libraries in Product like Format", PLOS ONE, vol. 10, no. 10, 15 October 2015 (2015-10-15), pages 1 - 16, XP055432449
- TURC HANINOVA ET AL.: "Pairing of T-cell receptor chains via emulsion PCR", EUROPEAN JOURNAL OF IMMUNOLOGY, vol. 43, no. 9, 1 September 2013 (2013-09-01), pages 2507 - 2515, XP055392257

Remarks:
The complete document including Reference Table(s) and the Sequence Listing(s) can be downloaded from the EPO website

(52) Cooperative Patent Classification (CPC): (Cont.)

C-Sets
C12Q 1/6806, C12Q 2531/113, C12Q 2563/159; C12Q 1/686, C12Q 2563/159

**Description**

**BACKGROUND**

The present disclosure relates to mining the antibody repertoire from pools of cells from a donor. In particular, the present invention relates to generating natively-paired scFv amplicons to enable screening for antibody binding and function.

Previous methods for mining the antibody repertoire from human donors have helped identify therapeutically valuable antibodies, define novel targets, and offer insight into the immune response to a disease. The methods for isolating these antibodies generally fall under two categories: isolating antibodies directly from cells such as B-cells or selecting antibodies from combinatorial libraries such as phage display, yeast display or mammalian display. The two approaches have different strengths; for example, antibodies obtained directly from B-cells usually have better potency and manufacturing properties while the display platforms offer the ability for subsequent screening, deep mining and clonal stability (Burton, D *et al.,* (2012), 12: 397-407). There is curently no high throughput technology that combines the benefits of both approaches.

Currently technologies are available to encapsulate large numbers of cells and subsequently sequence their $V_H$ and $V_L$ domains by next-generation sequencing technologies. The original pairing of variable domains are maintained by the use of barcoded primers on particles that are encapsulated along with the B-cells. These technologies enable phylogenetic analysis of the sequences without any information about the antigen specificity or other biological functions of the repertoire. These technologies do not enable high throughput translation and subsequent screening of the antibody sequences. This creates a serious limitation in the functional analysis of the immune repertoire (DeKosky, B.J. et al., Nat. Biotechnol. (2013), 31: 166-169; Stern J. Sci Trans. Med. (2014), 6: 248; Tan Y-C, Arthritis. Rheumatol. (2014), 66: 2706-2715; Tan Y-C, Clin. Immunol. (2014), 151: 55-65; Lu, D.R. Clin. Immunol. (2014), 152: 77-89; Robins, W.H. Curr. Opin. Immunol. (2013), 25: 646-652; DeKosky, B. J. et al., Nat. Med. (2015), 21: 86-91). Moreover, validating antibody leads requires gene synthesis, cloning and expression which can create a severe bottleneck in the number of candidates that can be functionally assessed (Galson, J. D., et al., Crit. Rev. Immunol. (2015), 35: 463-478).

Generating recombinant antibody fragments, such as scFv and Fab, from single cells in microtiter plates has been described, although this approach is severely limited in throughput, in that it can handle at most a few thousands of cells at a time, with a maximum success rate of 30-60% (Meijer, P. J. et al. J.Mol.Biol. (2006), 358: 764-772; Tiller, T. J Immunol. Methods. (2008), 329: 112-124).

Both of these technologies suffer from low screening throughput that overwhelmingly under-samples the $\sim 10^7$ B cells obtained from a typical blood draw.

Accordingly, there is a need in the art for a technology or approach that is able to rapidly isolate natively-paired antibody sequences from human donors at a high enough throughput to adequately cover the natural diversity and in a format that enables rapid screening for activity.

**PRIOR ART CITED IN THE EUROPEAN SEARCH OPINION**

Levin et al J Allergy Clin Immunol. 2016 May;137(5):1535-44 reltaes to the persistence and evolution of allergen-specific IgE repertoires during subcutaneous specific immunotherapy. Turchaninova et al Eur J Immunol. 2013 Sep;43(9):2507-15 relates to the pairing of T-cell receptor chains via emulsion PCR. DeKosky et al Nature Biotechnology 2013 31;166-169 relates to high-throughput sequencing of the paired human immunoglobulin heavy and light chain repertoire.

**SUMMARY OF THE DISCLOSURE**

The subject-matter of the invention is defined by the appended claims. Any further aspects of the disclosure that fall outside the claim-scope are provided for information only.

The present disclosure is concerned with a method for generating a library of natively-paired scFv amplicons that can be screened for antibody binding and function.

The present disclosure is also concerned with the use of microfluidics for encapsulating single cells in droplets.

**BRIEF DESCRIPTION OF THE FIGURES**

The present invention will now be described in more detail with reference to the attached Figures, in which are shown:

**Figure legends:**

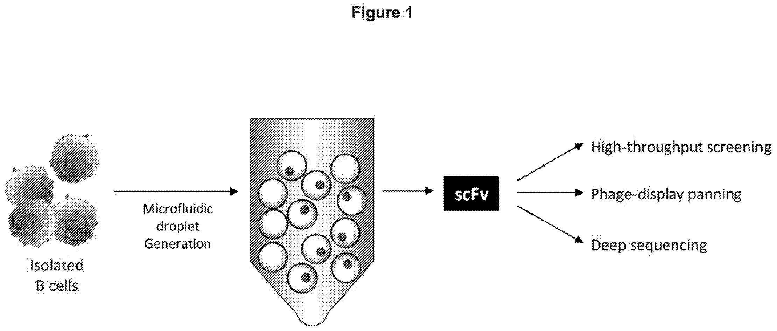

**Figure 1: Strategy for the Immune Replica platform.** Cells (*e.g.* B-cells) isolated from patients (*e.g.* convalescent patients) are encapsulated into water-in-oil droplets with RT-PCR reagents such that cognate $V_H$ and $V_L$ domains are amplified and linked. The resulting amplicon forms an expression-ready scFv which can be expressed as scFv-Fc or IgG for screening, displayed on phage for selections, or deep sequenced for repertoire characterization.

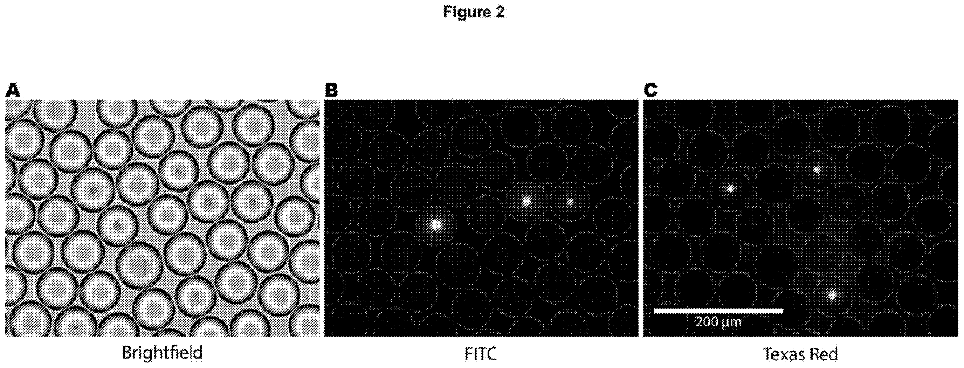

**Figure 2: Microfluidic encapsulation of single cells.** Microfluidic encapsulation of single cells stained red or green prior to encapsulation was visualized in the (A) Brighfield, (B) FITC and (C) Texas Red channels. Outlines of the droplets boundaries are overlaid on channels B and C for reference.

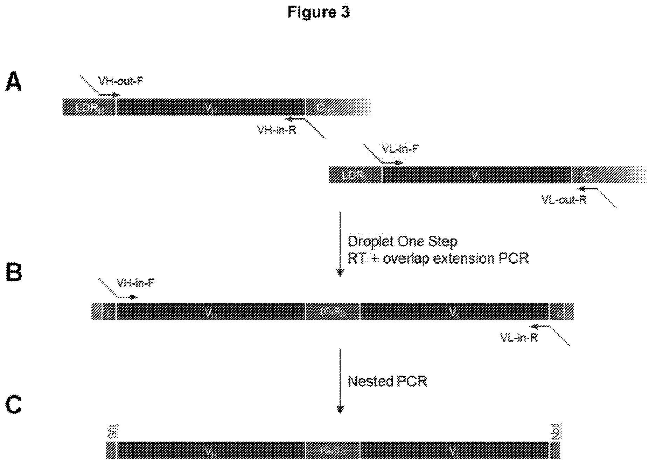

**Figure 3: Strategy for recovery of natively-paired scFv from primary B-cells. (A)** Cognate $V_H$ and $V_L$ domains are amplified within droplets using primer pools designed from IMGT germline sequences. The two amplicons are linked using primers with complementary overhangs (green), which also generate a $(Gly_4Ser)_3$ linker. By using limiting amounts of VH-in-R and VL-in-F primer pools, amplification of the full scFv over the individual $V_H$ and $V_L$ domains is favored. **(B)** A nested PCR step using primers specific to $V_H$ FR1 and $V_L$ FR4 amplify the final scFv **(C)** with overhangs that enable cloning via Sfi1/Not1 into vectors for panning or direct screening.

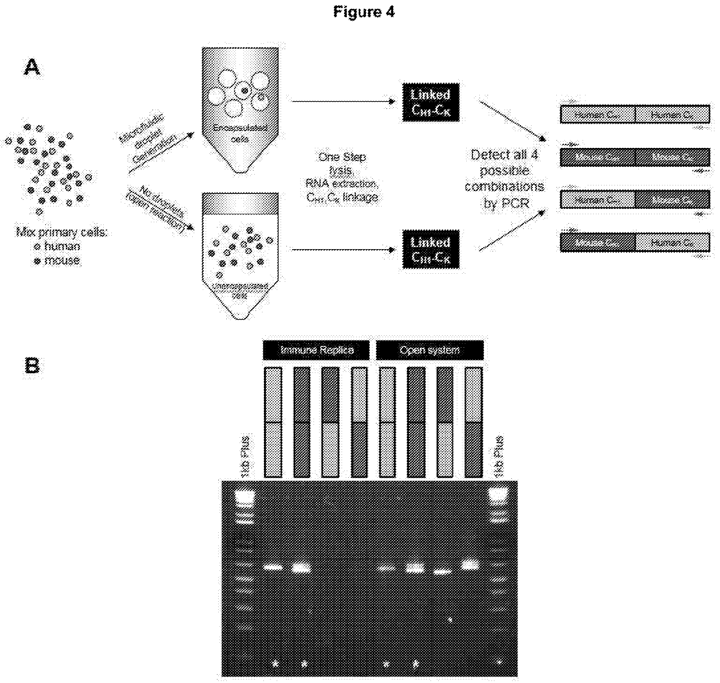

**Figure 4: Immune Replica platform maintains cognate chain pairing. (A)** Primary human and mouse B-cells were mixed and either encapsulated or treated in unencapsulated form ("open system"/"combinatorial"). Primers specific to constant regions were used to generate linked $C_{H1}$-$C_K$ amplicons and chain pairing was determined using primers specific to human (light gray) or mouse (dark gray) regions . **(B)** Agarose gel depicting amplification of correctly-paired species (indicated by asterisk) can be obtained using encapsulation, whereas the open system generates all possibilities in relatively equal amounts.

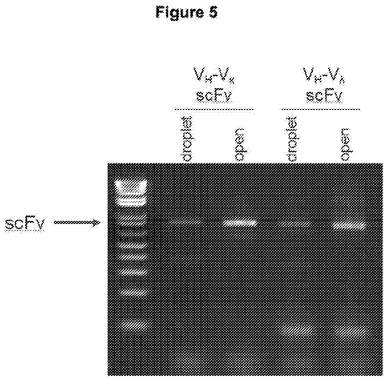

**Figure 5: Generation of scFv amplicons from primary B-cells.** Expression-ready scFv amplicons were generated from 48-hour stimulated human B-cells in either encapsulated ("droplet" - lanes 2 and 4) or unencapsulated ("open" - lanes 3 and 5) formats.

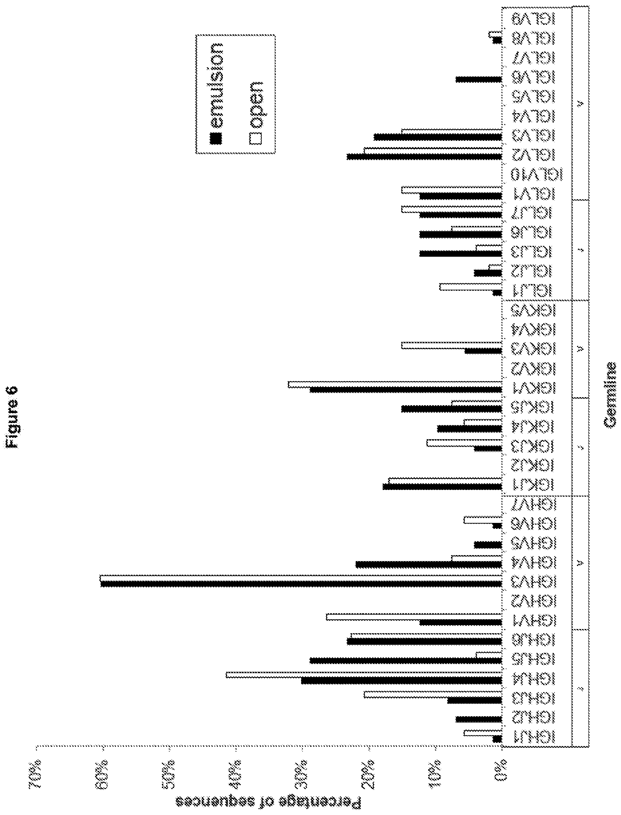

**Figure 6: Distribution of V/J germline diversity from deep sequencing.** ScFv amplicons were generated from two healthy individuals with or without encapsulation and used as template to amplify $V_H$, $V_\kappa$ and $V_\lambda$ domains which were barcoded and sequenced by paired-end Illumina sequencing. Unique sequences were aligned to IMGT germline sequences and are displayed as a percentage of total unique sequences obtained from droplet (black bars) or open (white bars) libraries .

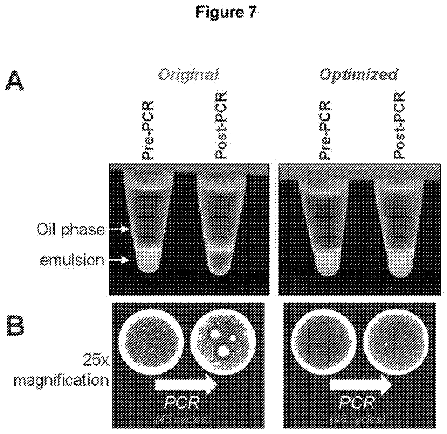

**Figure 7: Process improvements led to greater droplet stability during PCR. (A)** Pictures of collected emulsions before and after PCR cycling (45 cycles). Optimized PCR conditions and excipients contributed to increased stability, as did generating monodisperse droplets, as seen in **(B)**.

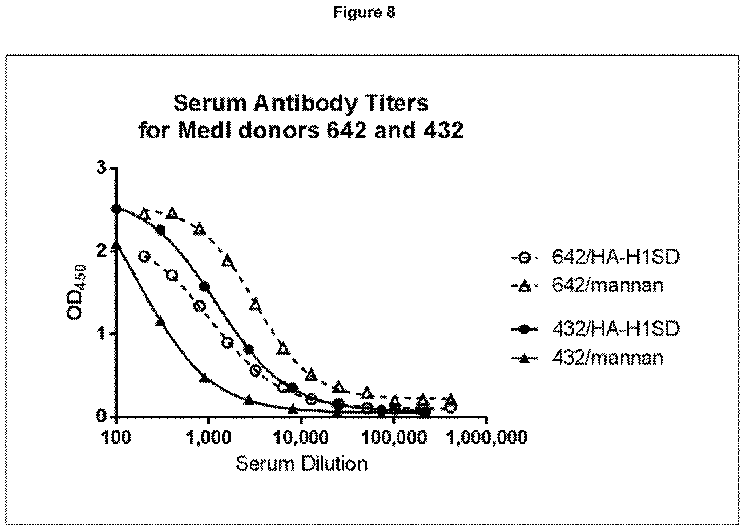

**Figure 8: Serum titers for two healthy donors (642 and 432) against Influenza hemagglutinin (HA-SD** - circles) and *C.albicans* **mannan (triangles).** Titers for donor 642 are shown in dashed lines/open symbols and those for donor 432 in solid lines/closed symbols.

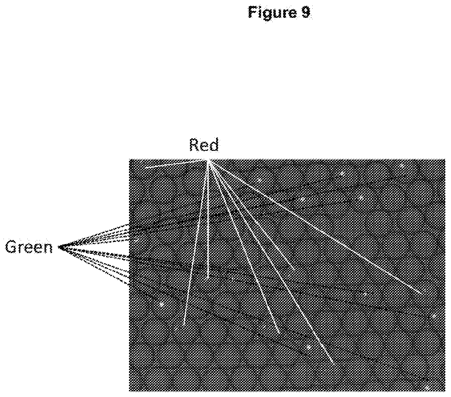

**Figure 9: Single-cell encapsulation using droplet microfluidics.** Cells were stained with CellTracker Red and Green dyes, mixed and encapsulated into droplets at a density that favored single-cell encapsulation. Each droplet forms an independent reaction vessel whereby that cell's cognate V genes can be amplified and paired. Scale bar = $400\mu$m.

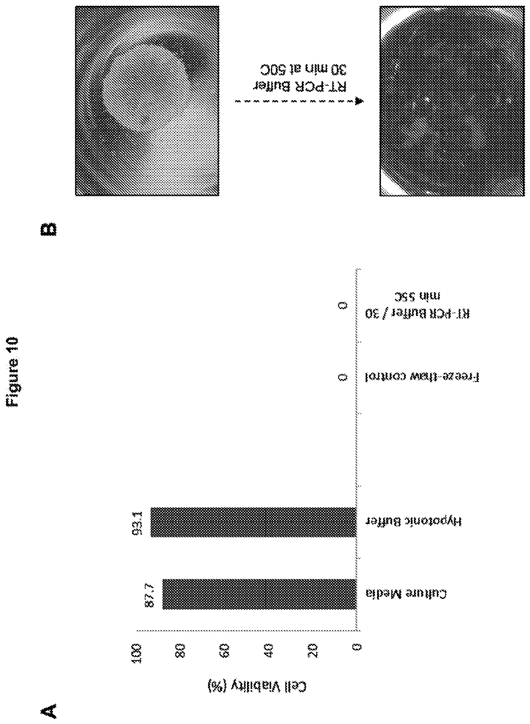

**Figure 10: Primary B cells are efficiently lysed during conditions of reverse transcription. (A)** Cell viability measurements were performed using the ViCell viability analyzer on cells incubated for 30 minutes in either culture medium, encapsulation buffer ("Hypotonic Buffer") and RT-PCR buffer. Viability after one freeze-thaw cycle was also measured for comparison. (B) Visual examination of cell lysis. Cells were stained with TrypanBlue and imaged before and after incubation with RT-PCR buffer for 30 minutes at 50°C.

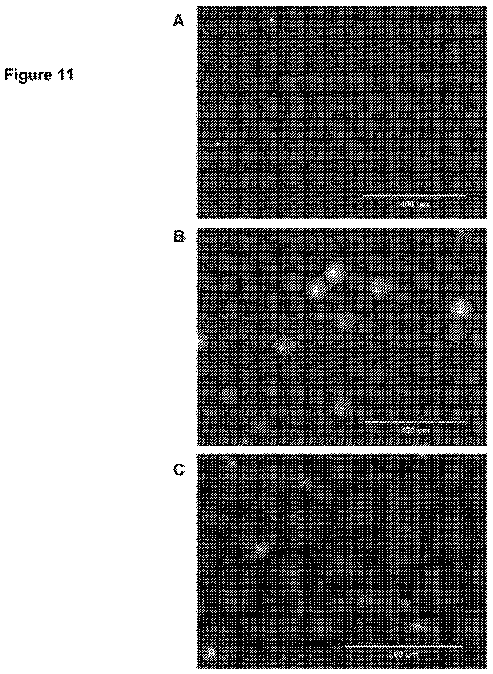

**Figure 11: Single cells are efficiently lysed within droplets with RT-PCR reagents.** IM9 multiple myeloma cells were stained with CellTracker Red or Green and encapsulated with PBS buffer (A) or RT-PCR buffer (B) shortly before imaging. (C) Single cells were encapsulated with RT-PCR reagents and SYBR-Green dye to visualize the release of double-stranded nuclear material upon cell lysis.

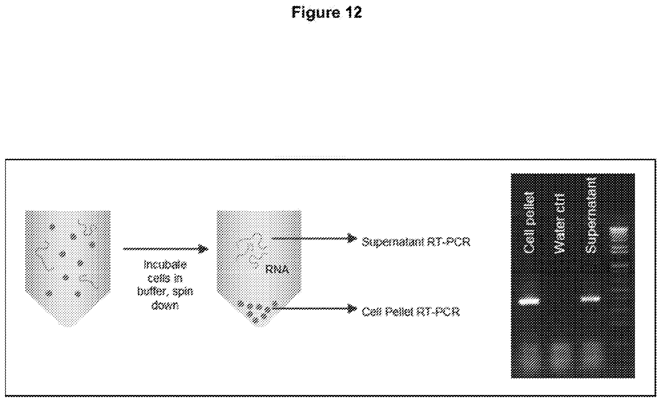

**Figure 12: Cell incubation results in free RNA being produced from dead or dying cells.**

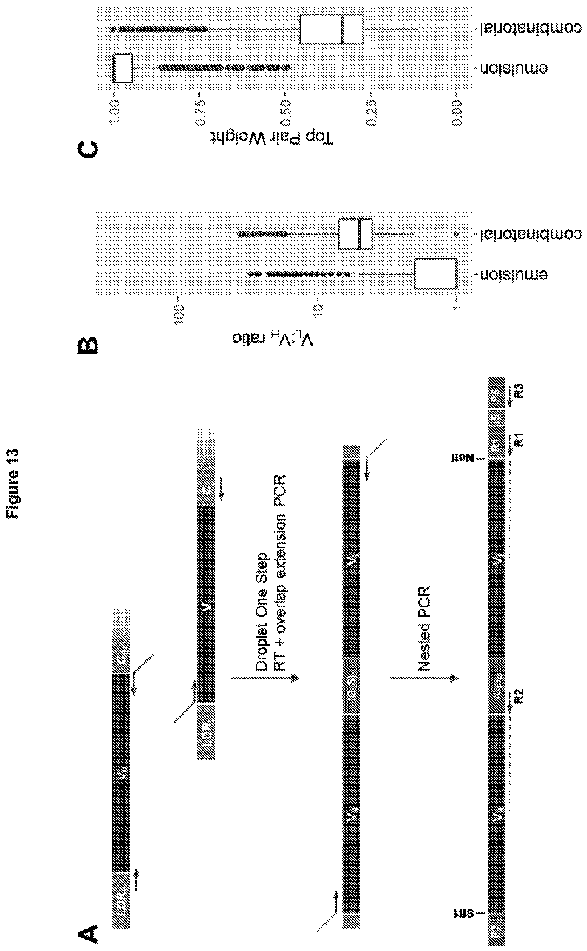

**Figure 13: Generating natively-paired libraries for screening and next generation sequencing (NGS). (A)** $V_H$ and $V_L$ domains from each encapsulated B cell are amplified with specific primer sets and paired in-frame via complementary overhangs. A nested PCR with $V_H$ FR1 and $V_L$ FR4 primers generates full-length scFv with overhangs to enable barcoded paired MiSeq sequencing. $V_H$ and $V_L$ FR3, CDR3 and FR4 are sequenced with R2 and R1 primers, respectively, while the R3 primer provides the index read to enable demultiplexing. The amplicons can be cleaved of adaptor sequences via Sfi1/Not1 restriction sites for subcloning into expression or phagemid vectors for downstream selection and screening. (B-C) NGS data from a representative dataset where scFv libraries from one million stimulated B cells were generated in either emulsion or combinatorial fashion. (B) Emulsion libraries overwhelmingly favored a 1:1 $V_L$:$V_H$ ratio (sample median) whereas combinatorial libraries were scrambled. (C) In the case where multiple pairings were seen, the dominant partner accounted for 99% of sequences in the emulsion library (sample median), whereas partners were more evenly distributed in the combinatorial library.

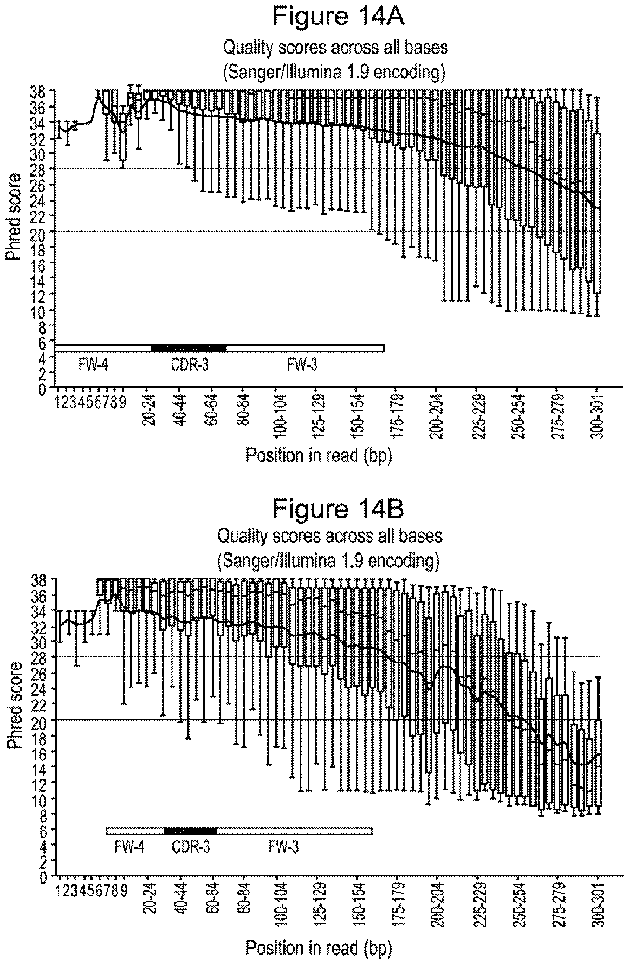

**Figure 14: Next-generation sequencing quality of paired $V_H$ and $V_L$ reads.** Representative sequencing Phred quality scores of (A) $V_H$ and (B) $V_L$ sequences. Framework (FW) and CDR regions are shown.

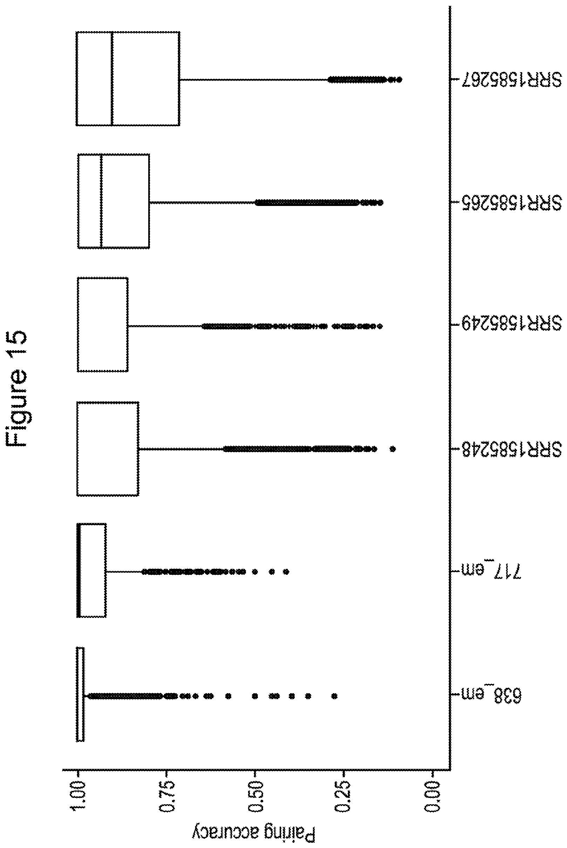

**Figure 15: $V_H$-$V_L$ pairing accuracy from top-pair analysis comparing antibody libraries generated within droplets from this study (lanes 1-2) and a reference study (**DeKosky, B.J. et al., Nat. Med. (2015), 21: 86-91**) (lanes 3-6).** Cell number and sequencing depth are comparable between samples. *** p<0.001 between Donor 1 or Donor 2 and any of the other libraries using a Student's t-test.

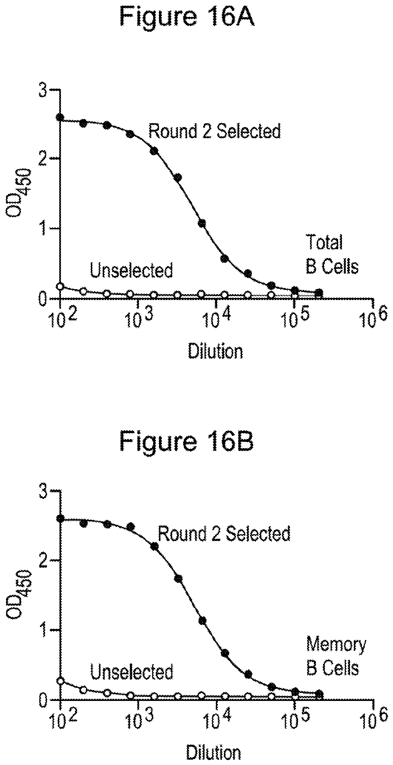

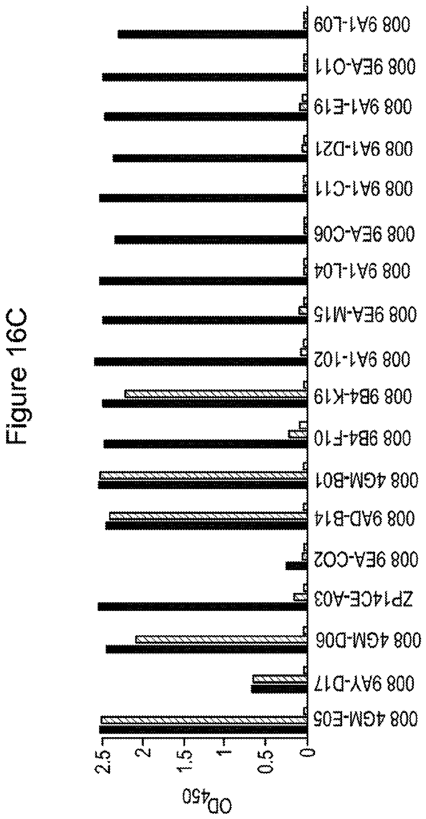

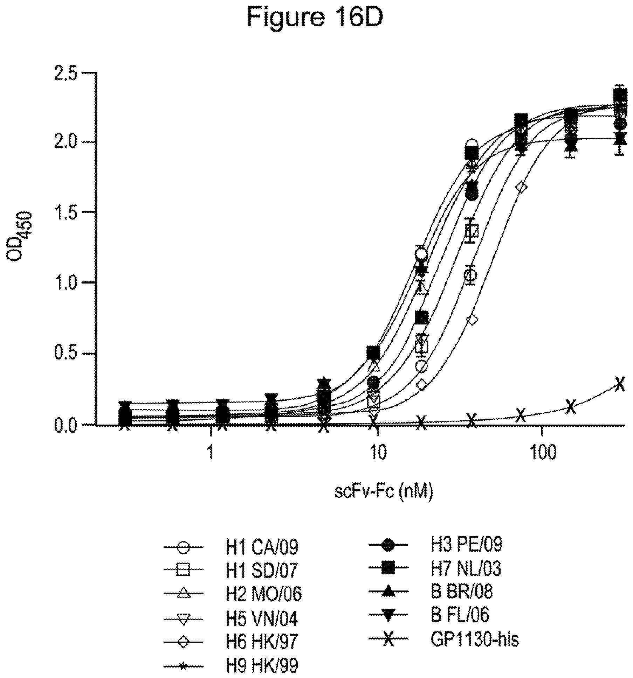

**Figure 16: Phage-display enrichment for specific binders.** Emulsion Libraries obtained from total (A) and memory (B) B cells were subjected to 2 rounds of phage display panning on hemagglutinin H1 (A/California/07/2009 H1N1) and enrichment was measured by polyclonal phage ELISA. (C) Specific ELISA binding data for 18 monoclonal scFv-Fc antibodies against hemagglutinin H1 (A/California/07/2009 H1N1 - solid), H5 (A/Vietnam/1203/2004 H5N1 - hatched) or an irrelevant control (white). (D) ELISA binding data showing cross-reactivity of one monoclonal scFv-Fc antibody against a panel of hemagglutinin antigens from Group 1 (blue) and 2 (red) subtypes of influenza A and both lineages of influenza B (green). Binding to a negative control his-tagged protein is shown in grey. Error bars represent the standard deviation of measurements performed in triplicate.

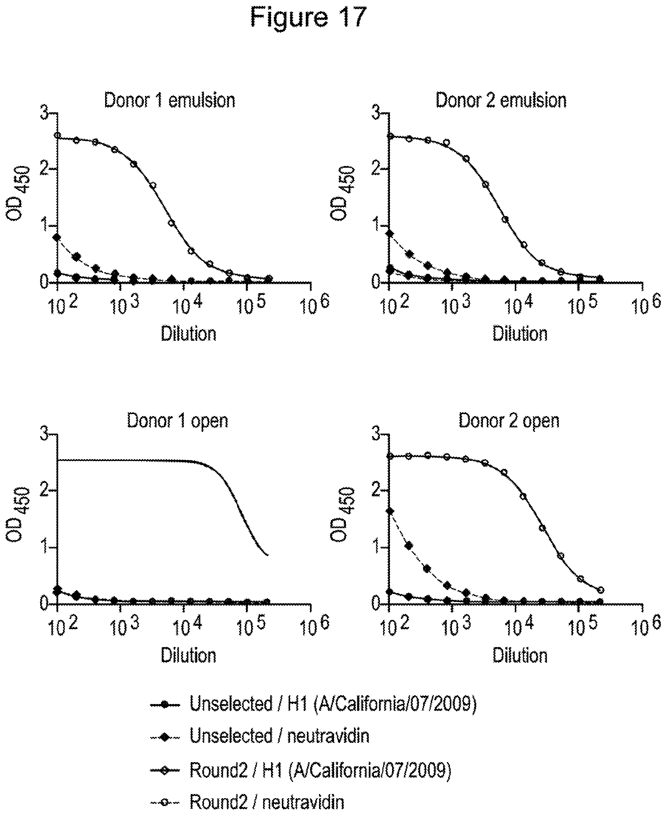

**Figure 17: Polyclonal phage ELISA showing enrichment after two rounds of selection on hemagglutinin H1.** Unselected library is shown for H1 CA/09, H1 SD/07, H2 MO/06, H5 VN/04, H6 HK/97 and H9 HK/99 , whereas the round 2 selected library is shown for H3 PE/09 and H7 NL/03. Binding of diluted phage was measured against H1 (solid lines) or an irrelevant protein control (dashed lines) for libraries generated from emulsified total B cells (A), emulsified memory B cells (B), combinatorial total B cells (C) or combinatorial memory B cells (D).

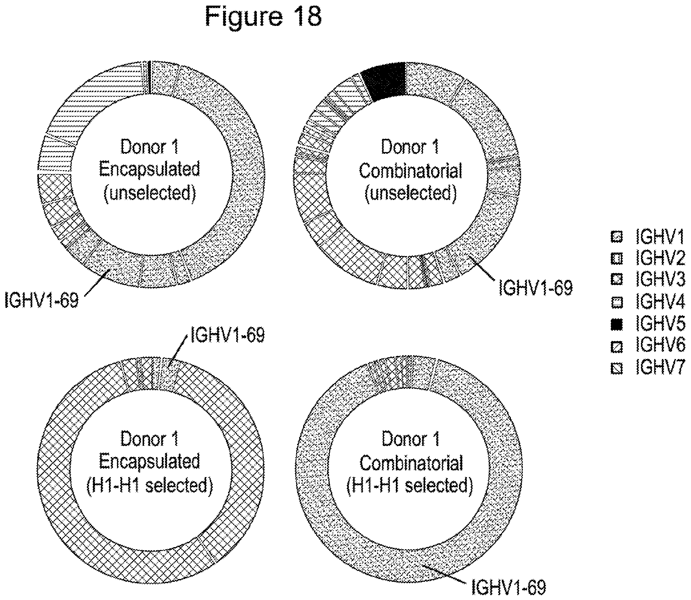

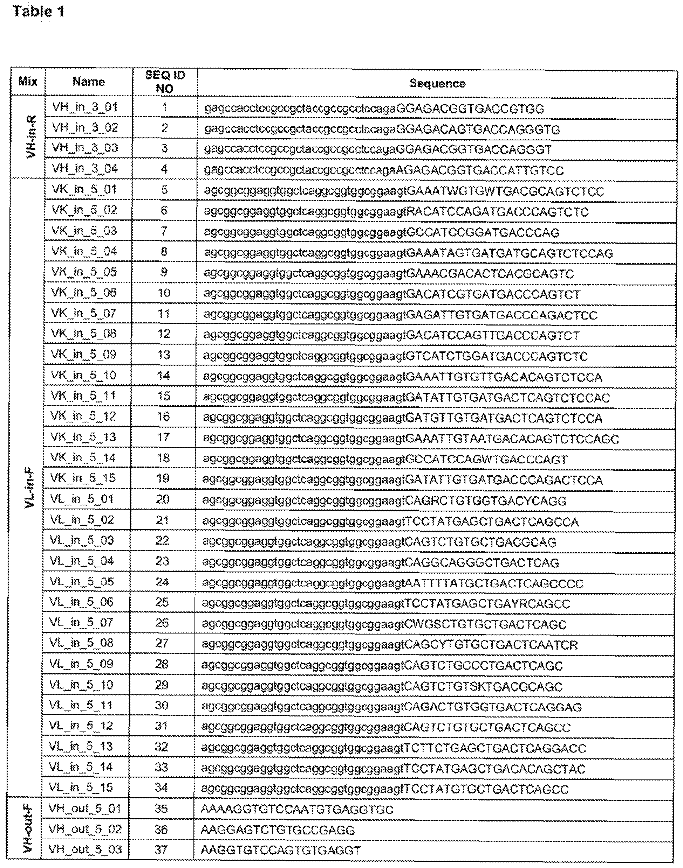

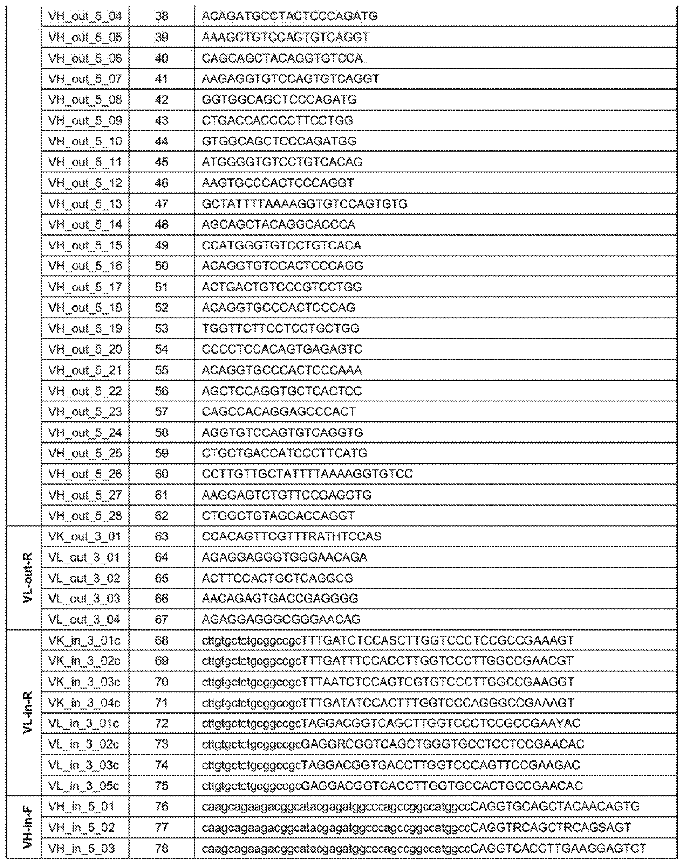

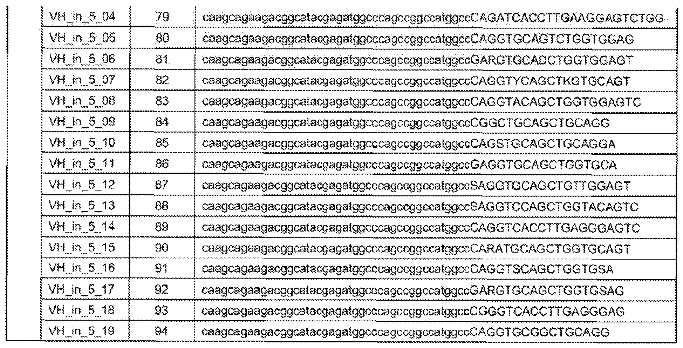

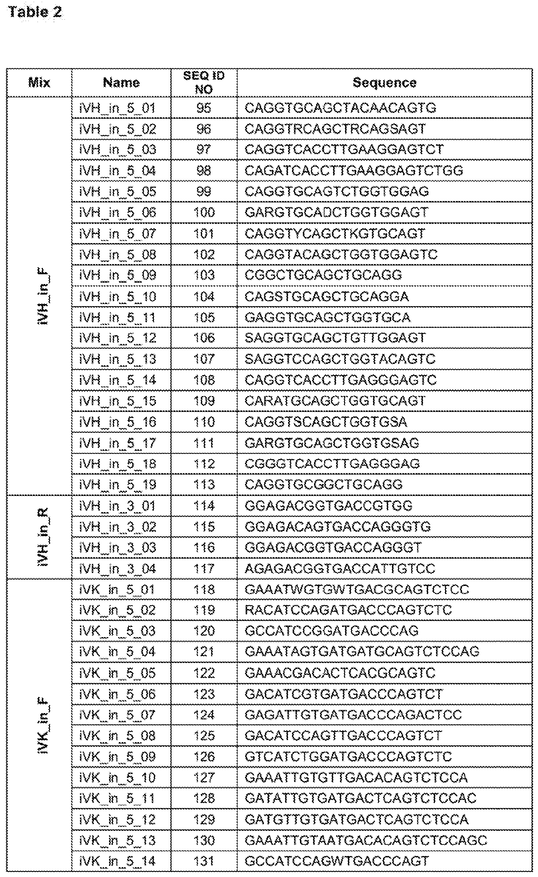

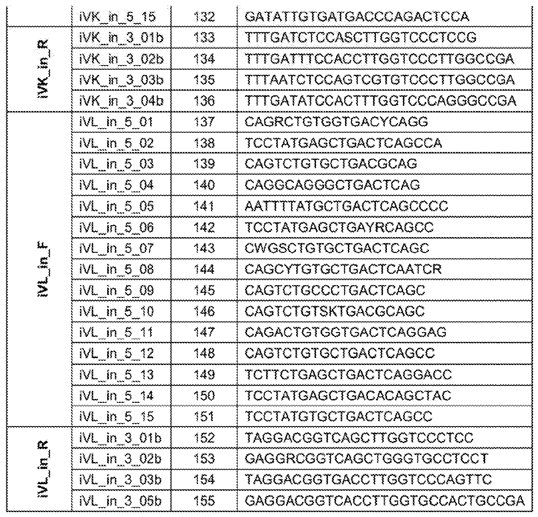

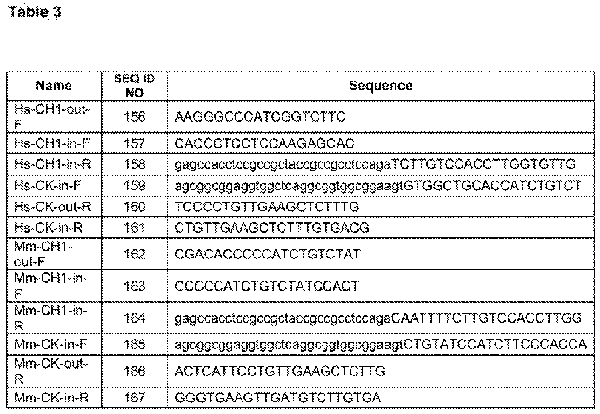

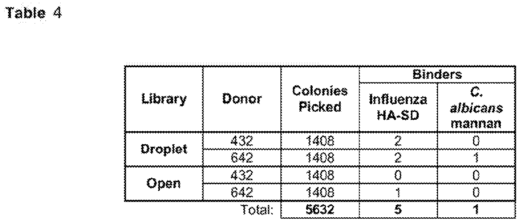

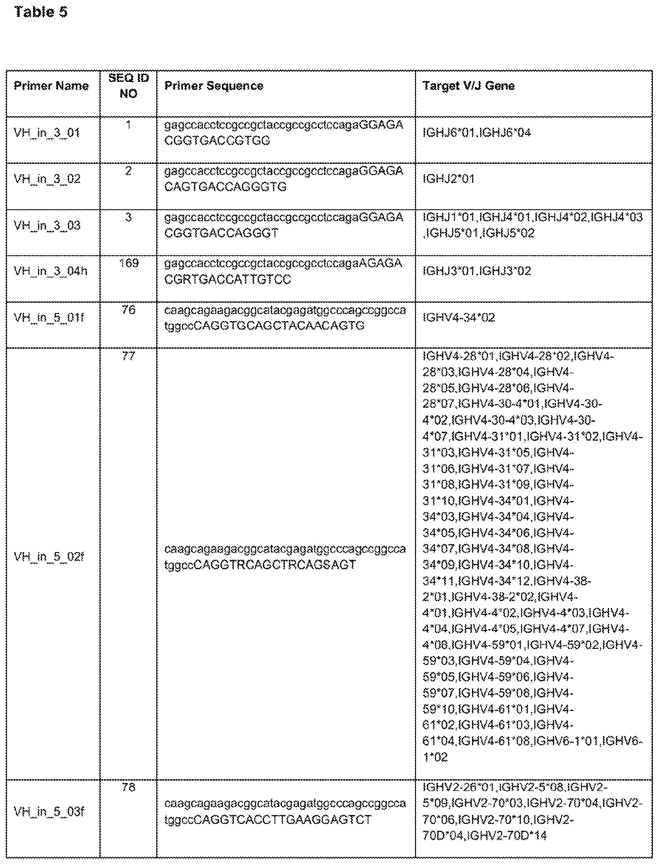

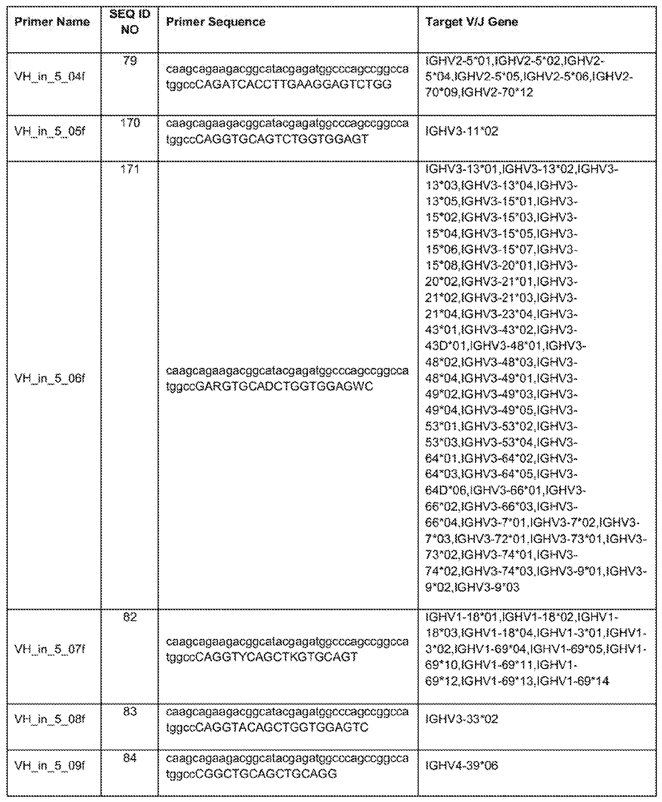

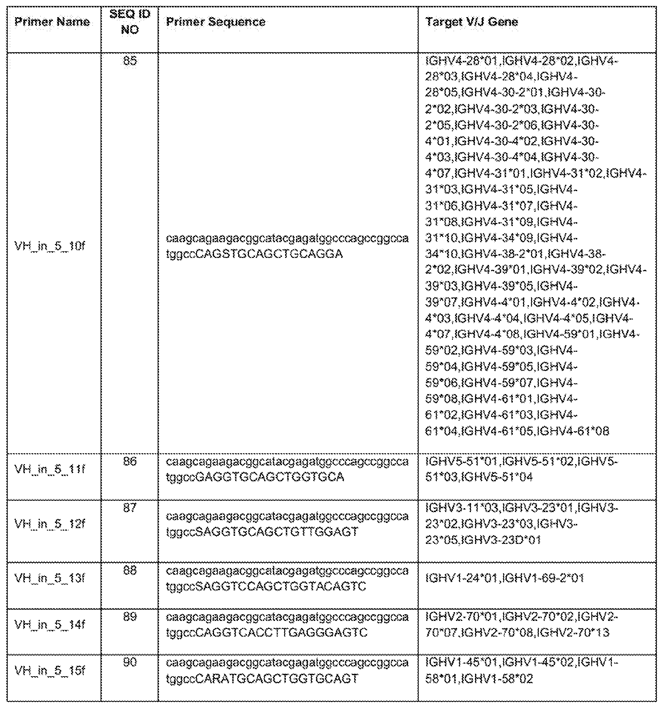

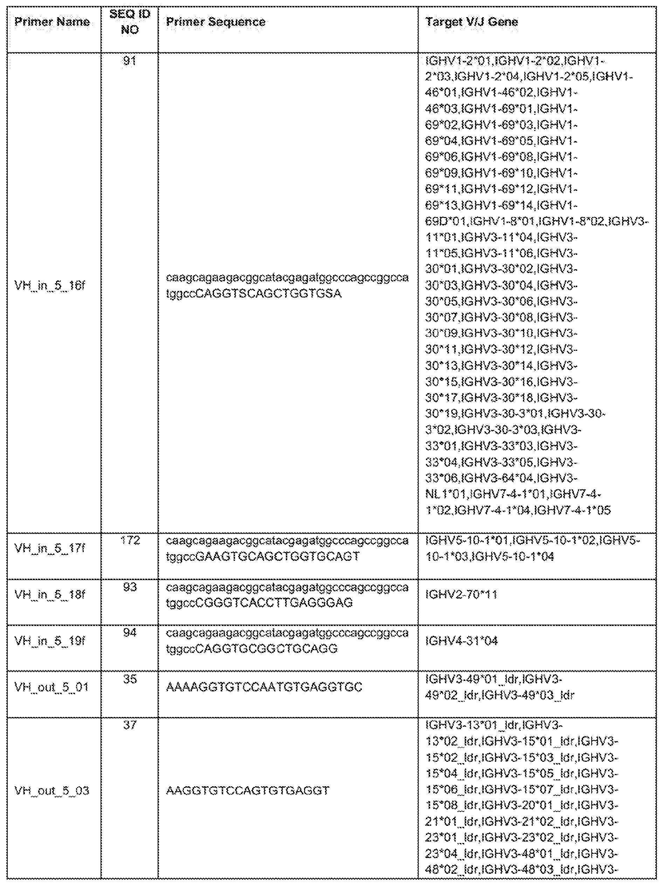

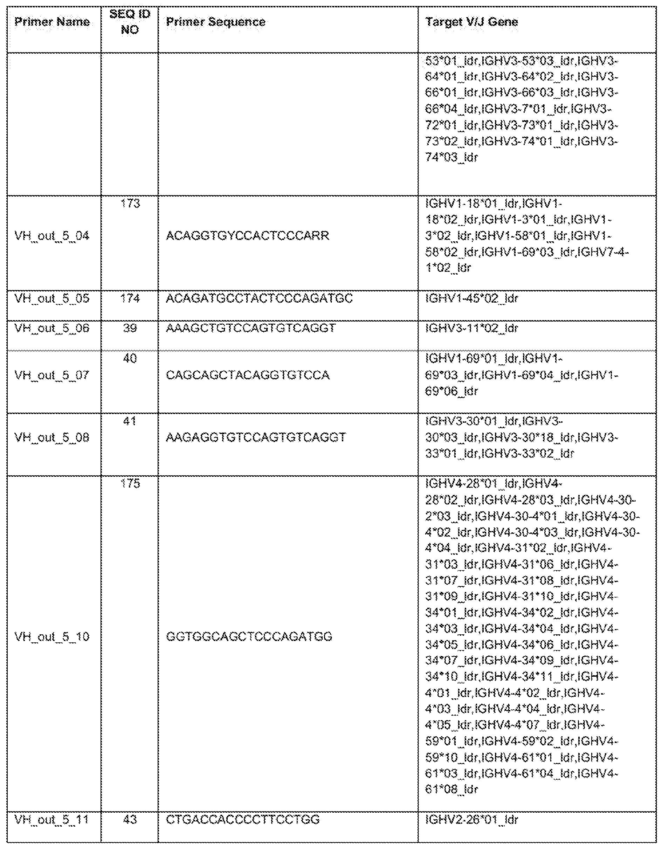

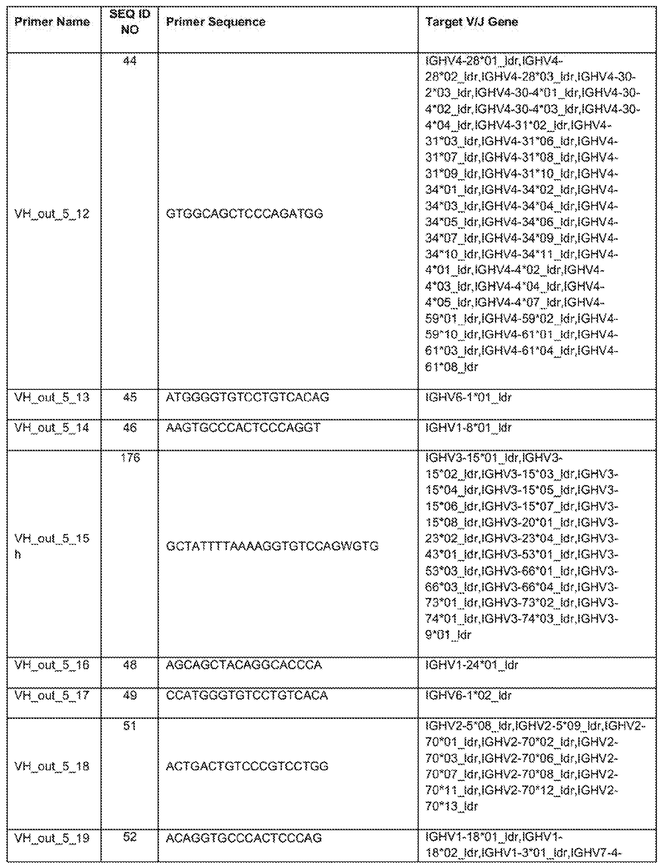

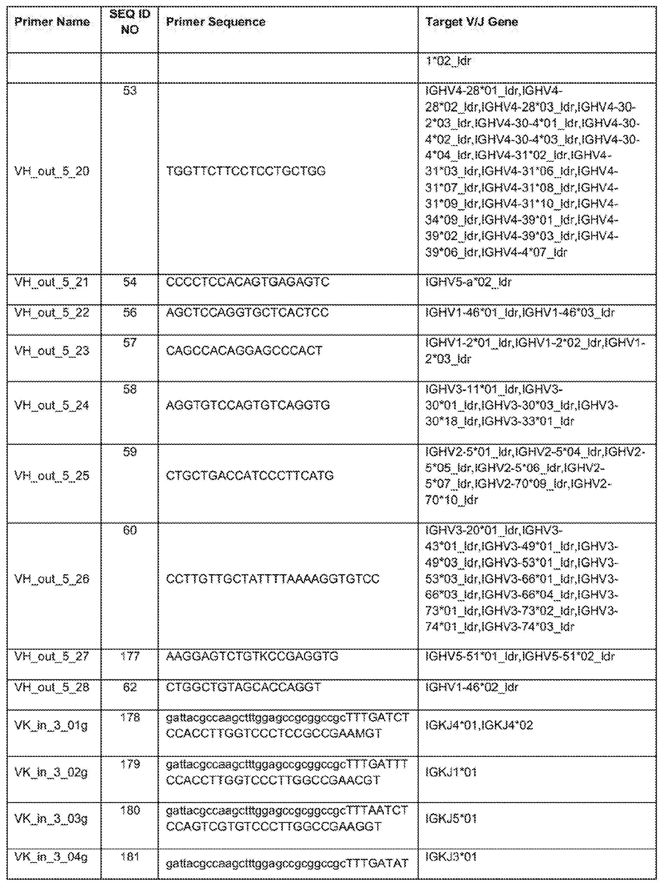

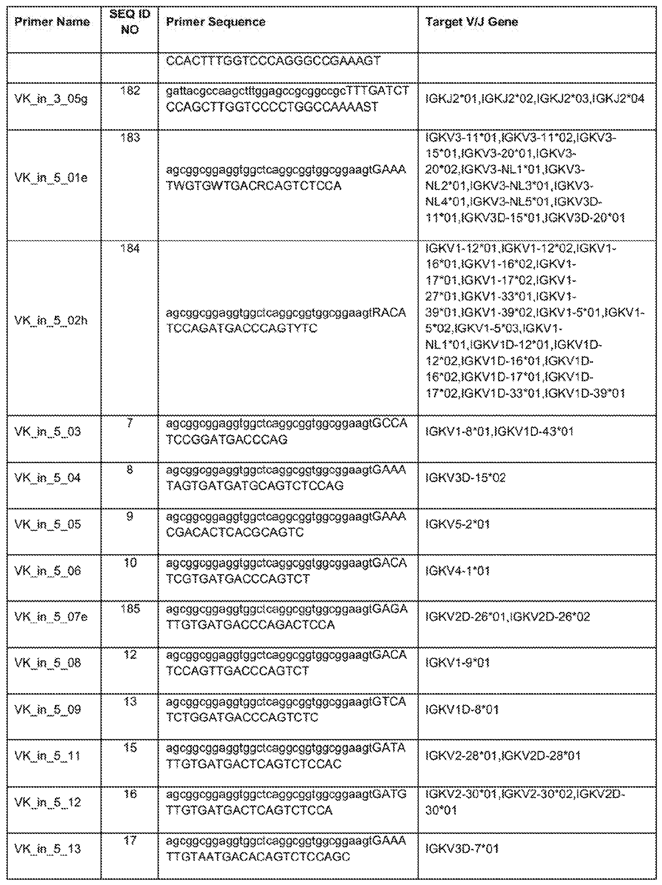

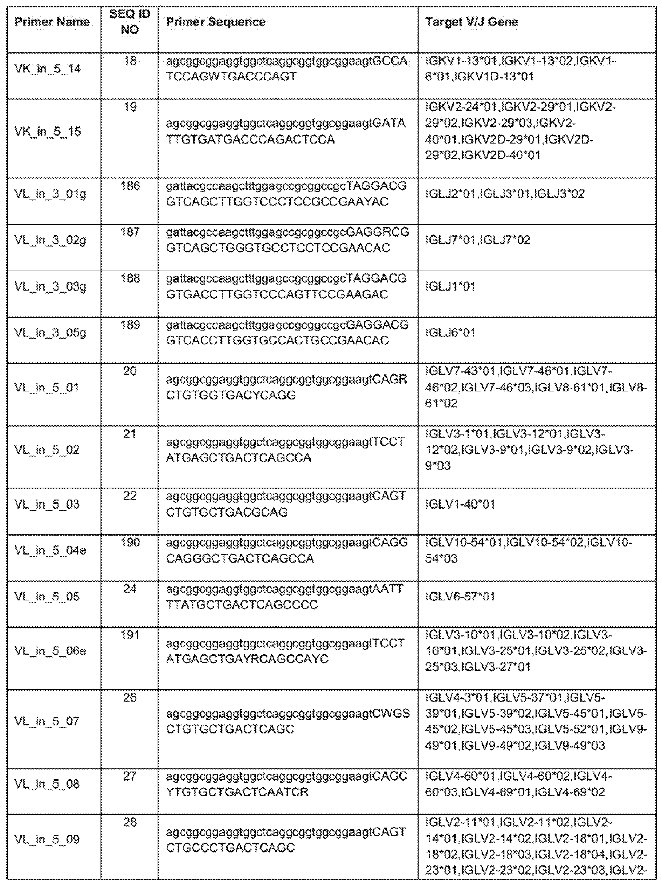

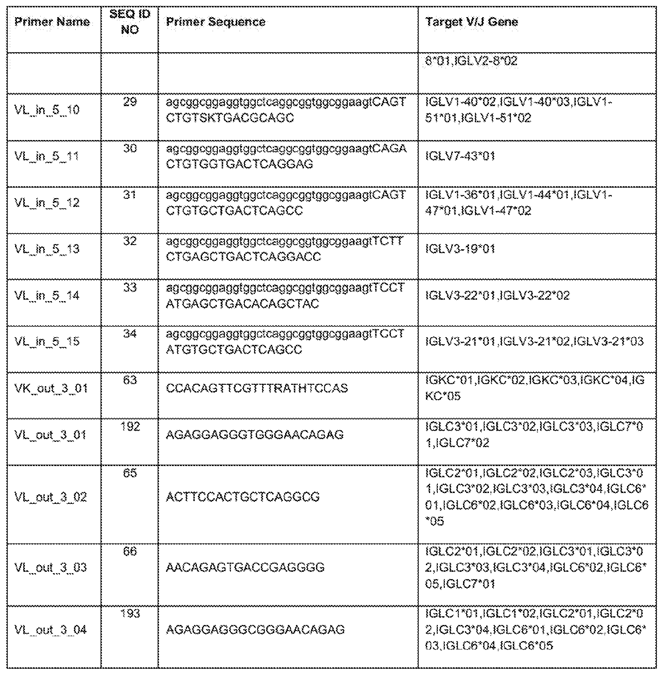

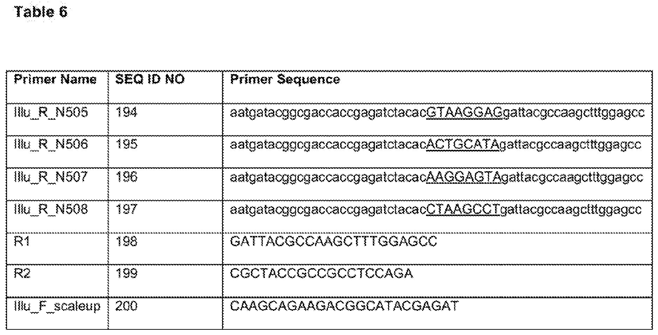

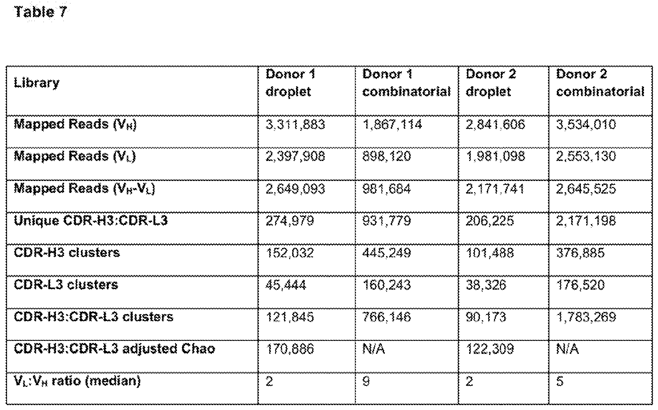

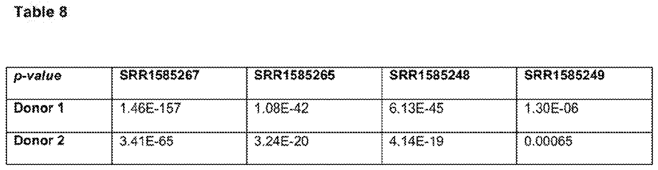

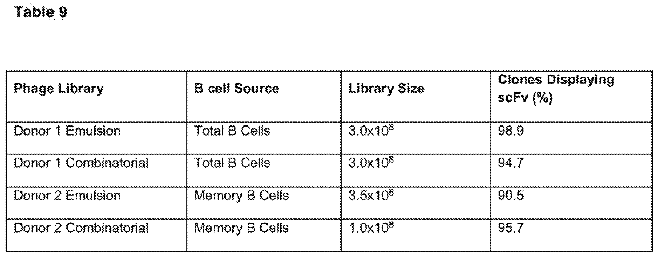

**Figure 18: Monoclonal VH germline distribution before and after 2 rounds of panning on hemagglutinin H1 (A/California/07/2009 H1N1).** Libraries from the same donor were generated in either encapsulated (A, C) or combinatorial (B, D) formats.

Unselected library distributions (A, B) were calculated from next-generation sequencing by mapping read clusters to individual VH germline genes. Over 100 clones were sequenced by Sanger sequencing to obtain the germline diversities following phage display selection. The relative abundance of IGHV1-69, a gene that encodes antibodies known to contact group 1 hemagglutinin through heavy chain interactions alone, is indicated within each plot.

**Table 1: Primer sequences used for scFv generation.**

**Table 2: Primers used for Illumina sequencing.**

**Table 3: Primers used for human and mouse $C_{H1}$/$C_K$ linking.**

**Table 4: Immune Replica campaign against two commonly found antigens.** B-cells from two healthy donors (642 and 432) were used for scFv generation. $V_H$/$V_L$ domains from -10,000 primary B-cells were amplified and paired in either encapsulated ("Droplet") or unencapsulated ("Open") formats to generate scFv cassettes that were directly cloned into an scFv-Fc expression vector. 5,632 bacterial-expressed scFv-Fc clones were screened by ELISA for binding to Influenza hemagglutinin (HA-SD) and *C.albicans* mannan.

**Table 5:** Primers used for V/J gene amplification. Regions of the primer that specifically bind the target gene are shown in uppercase whereas overhangs are shown in lowercase. Target V/J genes are listed, with the leader sequence for each gene designated by the suffix "_ldr".

**Table 6: Primers used in library amplification for phage display generation or next generation sequencing profiling.** Specific index sequences are underlined.

**Table 7: Analysis of unique sequences from next-generation sequencing data.**

**Table 8: Statistical comparison of $V_H$-$V_L$ pairing accuracy from top-pair analysis using Student's t-test.** Antibody libraries generated within droplets are compared between this study (Donor 1 and Donor 2) and a reference study (DeKosky, B.J. et al., Nat. Med. (2015), 21: 86-91) (datasets SRR1585248, SRR1585249, SRR1585265 and SRR1585267). Cell number and sequencing depth are comparable between samples.

**Table 9: Phage display library characterisation.**

## DETAILED DESCRIPTION

The present disclosure permits encapsulation of single cells isolated from patients, e.g. B-cells, in water-in-oil droplets, with reagents to amplify and link native pairings of heavy and light chain variable domain amplicons from single encapsulated cells, in order to create a recombinant library of scFv. Throughout the description reference is made to phage display, but it will be appreciated by the person skilled in the art that yeast display and mammalian display technologies are equally applicable, and the inventors have explicitly contemplated such alternative display systems.

The present disclosure involves cloning the variable domains ($V_H$ and $V_L$) from single encapsulated cells and joining them to form an scFv. By physically separating each cell, native $V_H$-$V_L$ pairing, which is critical to recovering antibody binding and function, is preserved. The resulting amplicon forms an expression-ready scFv. The library of scFv is a translatable scFv library that can either be directly screened for binding and function, enriched by phage-display panning, or deep-sequenced using next-generation sequencing.

The present disclosure further permits coupling of the expression-ready scFv library with the screening methods (e.g. phase-display) to enrich for antigen-specific clones. The present disclosure allows the high throughput identification of antigen-specific antibodies, in particular by mining the natural B-cell diversity to rapidly isolate antigen-specific antibodies from human patients. The present invention allows the identification of antibodies that are not found by existing technologies.

The present disclosure provides a method for producing encapsulated natively-paired scFv amplicons, the method comprising: encapsulating single B-cells in droplets, wherein the droplets further contain reagents for amplifying and linking native pairings of heavy and light chain variable domain amplicons from single encapsulated cells; lysing the single encapsulated cells and generating the encapsulated natively-paired scFv amplicons, wherein each scFv amplicon comprises a native pairing of heavy and light chain variable domain amplicons linked together by a linker, wherein the linker has a length of 10-20 amino acids.

Typically the scFv amplicon comprises the formula V1-L-V2. L is the linker. In one instance, V1 is the heavy chain variable domain and V2 is the light chain variable domain i.e. the scFv amplicon has the formula VH-L-VL. In another instance, V2 is the heavy chain variable domain and V1 is the light chain variable domain i.e. the scFv amplicon has the formula VL-L-VH.

In one instance, the reagents for amplifying and linking native pairings of heavy and light chain variable domain amplicons as defined anywhere herein comprise primers designed to human Ig sequences. In one instance, the reagents for amplifying and linking native pairings of heavy and light chain variable domain amplicons as defined anywhere herein comprise a primer pool comprising the primers as set out in Table 1. In one instance, the reagents for amplifying and linking native pairings of heavy and light chain variable domain amplicons as defined anywhere herein comprise a primer pool comprising the primers as set out in Table 5. The group of primers in Table 1 or Table 5 may be further subdivided if desired, for instance, appropriate sets of primers may be used where the cells are separated into kappa or lambda expressing cells or into cells expressing different Ig H isotypes. Primers may be designed with custom software known in the art. Heavy and light chain variable domain amplicons are initially formed from the native heavy and light chain variable domain sequences in the generation of a scFv amplicon.

In one instance, the generating step for generating the encapsulated scFv amplicons as defined anywhere herein comprises initially forming heavy and light chain variable domain amplicons from native heavy and light chain variable domain sequences and the reagents comprise a primer pool comprising first and second heavy chain variable domain primers; and first and second light chain variable domain primers, wherein the first heavy chain variable domain primer and the first light chain variable domain primer interact to join the heavy and light chain variable domain amplicons. In one instance, the primer pool comprises a lower concentration of the first primers than the second primers. Preferably, the concentration of the first primers is reduced by a factor of between two and eight, e.g. two, three, four, five, six, seven, eight, nine or ten, compared to the concentration of the second primers. Preferably, the concentration is reduced by a factor of eight. By providing a limiting amount of the nucleic acid primers that bind inside the variable domains, amplification of the full scFv is favoured over the individual $V_H$ and $V_L$ domains.

In one instance, the first heavy chain variable domain primer as defined anywhere herein is fused to a first overhang sequence and the first light chain variable domain primer as defined anywhere herein is fused to a second overhang sequence, wherein the overhang sequences interact to join the heavy and light chain variable domain amplicons. Preferably, the first and second overhang sequences are at least partially complementary. More preferably, the first and second overhang sequences are fully complementary. The two domain amplicons may be linked using overlap-extension PCR to generate a scFv amplicon.

In one instance, the RT-PCR is used in combination with overlapping-extension PCR.

In one instance, the first heavy chain variable domain primer as defined anywhere herein is the reverse primer which binds inside (typically at the 3' terminus of FR4) the heavy chain variable domain of the native sequence/amplicon, and the second heavy chain variable domain primer as defined anywhere herein is the forward primer which binds outside the heavy chain variable domain of the native sequence/amplicon; and the first light chain variable domain primer as defined anywhere herein is the forward primer which binds inside (typically at the 5' terminus of FR1) the light chain variable domain of the native sequence/amplicon, and the second light chain variable domain primer as defined anywhere herein is the reverse primer which binds outside the light chain variable domain of the native sequence/amplicon.

In another instance, the first heavy chain variable domain primer as defined anywhere herein is the forward primer which binds inside (typically at the 5' terminus of FR1) the heavy chain variable domain of the native sequence/amplicon, and the second heavy chain variable domain primer as defined anywhere herein is the reverse primer which binds outside the heavy chain variable domain of the native sequence/amplicon; and the first light chain variable domain primer as defined anywhere herein is the reverse primer which binds inside (typically at the 3' terminus of FR4) the light chain variable domain of the native sequence/amplicon, and the second light chain variable domain primer as defined anywhere herein is the forward primer which binds outside the light chain variable domain of the native sequence/amplicon.

These reagents allow the cognate $V_H$ and $V_L$ domains to be amplified within droplets.

The present disclosure also permits encapsulation of single cells isolated from patients. In one instance, the encapsulating step as defined anywhere herein comprises using microfluidics. Microfluidics is able to capture millions of cells, potentially the entire repertoire, into picoliter-sized droplets for parallel amplification into a library and thus provides a high throughput approach. In one instance, the library is a scFv library (Figure 1).

In one instance, the microfluidics as defined anywhere herein comprises using a glass microfluidic chip with pressure pumps. In one instance the microfluidic chip as defined anywhere herein is a fluorophillic chip. The microfluidic chip is designed to merge two streams of aqueous fluids: one carrying a suspension of cells and the other containing reagents for one-step reverse transcription (RT) and overlap-extension PCR. Microfluidics is used to reliably generate evenly sized droplets at high rates.

Though it has been reported by several groups that cell-based RT-PCR is not feasible in volumes of less than 5 nL (DeKosky, B.J. et al., Nat. Biotechnol. (2013), 31: 166-169; DeKosky, B.J. et al., Nat. Med. (2015), 21: 86-91; White, A. K. et al., Proc. Natl. Acad. (2011), 108: 13999-14004; Eastburn, D. J. et al., PloS ONE (2013), 8: e62961), the method of the disclosure is able to successfully amplify Ig transcripts directly from cells in picoliter-sized droplets *e.g.* droplets of 200pL in volume. In one instance, the droplets as defined anywhere herein are from about 50 pL to about 600 pL in volume. In one instance, the droplets are from about 100 pL to about 300 pL in volume. In one instance, the droplets are about 200 pL in volume.

In one instance, the encapsulating step as defined anywhere herein comprises combining an aqueous suspension with an oil to form an emulsion comprising the encapsulated single cells in water-oil droplets, wherein the aqueous suspension comprises the cells and the reagents for amplifying and linking native pairings of amplicons.

In one instance, the method further comprises a step prior to the encapsulating step, the step comprising providing the aqueous suspension comprising the cells and the reagents for amplifying and linking native pairings of amplicons. In one instance, the providing step comprises stimulating the cells in a first aqueous suspension comprising the cells and subsequently combining the cells with the reagents for amplifying and linking native pairings of amplicons to form the aqueous suspension comprising the cells and the reagents for amplifying and linking native pairings of amplicons. It is generally understood that the cells may be stimulated for about 48 hours, preferably at least 48 hours.

Titration of the cell suspension achieves approximately 1 cell for every 10 droplets which, based on Poisson statistics, results in single-cell encapsulation with >95% probability (Figure 9). In one instance, the suspension of cells as defined anywhere herein is at a density of about 1 to about 5 million cells/ml. In one instance, the suspension of cells as defined anywhere herein is at a density of about 3.5 to about 4.5 million cells/ml. In a more preferred instance, the suspension of cells as defined anywhere herein is at a density of about 4 million cells/ml. These densities are optimal for obtaining single-cell encapsulation into droplets (Figure 2). These densities also obtain single-cell encapsulation into droplets approximately 100 $\mu$m in diameter. Empty droplets may also be generated, although these do not contribute to the library since no template cells are present.

In one instance, the suspension of cells as defined anywhere herein comprises an anti-clumping agent. In another instance, prior to encapsulation, the suspension of cells as defined anywhere herein is stirred *e.g.* with a paramagnetic stir disk. Use of an anti-clumping excipient and/or stirring prevents suspended cells from settling prior to encapsulation. Since stimulated cells, *e.g.* B-cells or T-cells, have a tendency to aggregate over time, use of an anti-clumping excipient and/or stirring prevents changes in flow rates, as well as multiple cells being encapsulated together.

In one instance, the suspension of cells as defined anywhere herein comprises a stabilizing agent. Preferably the stabilizing agent is an amphipathic molecule. More preferably, the stabilizing agent is acetylated BSA. Acetylated BSA is an amphipathic molecule which can stabilize the water-oil interface and lower the interfacial tension (Dalgleish, Trends in Food Science & Technology (1997), 8 (1): 1-6). The present inventors have determined that use of acetylated BSA decreases droplet coalescence during the harsh conditions of PCR cycling. Further, the presence of acetylated BSA may protect enzymes such as reverse transcriptase from denaturation at the interface.

In one instance, the generating step for generating the encapsulated amplicons as defined anywhere herein comprises RT-PCR.

In one instance, the oil as defined anywhere herein is a low viscosity oil. In a preferred instance, the oil as defined anywhere herein is fluorinated oil. In a more preferred instance, the oil as defined anywhere herein is HFE-7500 fluorinated oil + 2% w/v 008-fluoro-surfactant (RAN Biotechnologies cat no 008-FLUOROSURFACTANT-HFE7500. Due to the life span of cells used in the present disclosure, the throughput of the present method is limited by the time it takes to encapsulate the cells. The present inventors have determined that throughput can be improved by reducing the viscosity of the oil used to form the water-oil droplets for encapsulating the cells. Specifically, the present inventors have determined that a less viscous oil allows greater flow rates. In one instance, the microfluidic flow rate for the oil is between 90 and 125 $\mu$L/min , preferably 100 $\mu$L/min and the aqueous fluid is between 5.6 and 7 $\mu$L/min, preferably 6.22 $\mu$L/min. Reducing the viscosity of the oil is preferred over alternatives such as increasing cell density because it reduces the risk of more than one cell being encapsulated in a single droplet.

The methods of the invention as defined above, allow millions of cells to be encapsulated. Preferably, at least 1, at least 2, at least 3, at least 4, at least 5, at least 6, at least 7, at least 8, at least 9 or at least 10 million cells are encapsulated. The methods of the disclosure as defined above, allow one million cells to be encapsulated within about 40 minutes.

In one instance, the reagents for amplifying and linking native pairings of amplicons as defined anywhere herein comprise standard RT-PCR reagents. In one instance, the reagents as defined anywhere herein comprise Titan (Roche cat no 11855476001).

The above optimization of the aqueous components and microfluidic flow rates generates droplets that have improved homogeneity in size and improved integrity during RT-PCR. This allows improved reliability in the generating amplicons.

Prior to the cells being encapsulated in droplets, some cells die and lyse, releasing their genetic material (e.g. nucleic acid) into the surrounding media. The nucleic acids, e.g. those encoding VH or VL domains, may contaminate droplets and lead to non-natively paired products. It will be appreciated by the person skilled in the art that it is desirable to minimize the levels of contaminating nucleic acid present in the droplets. In particular, the free nucleic acid from dead or dying cells is RNA. In one instance, the method as defined anywhere herein further comprises preventing at least some free nucleic acid from dead or dying cells from being encapsulated in droplets. In one instance, the method as defined anywhere herein further comprises preventing substantially all free nucleic acid from dead or dying cells from being encapsulated in droplets. Preferably, the method as defined anywhere herein further comprises preventing free nucleic acid from dead or dying cells from being encapsulated in droplets. It will be appreciated by the person skilled in the art that any method for achieving this is within the scope of the present disclosure.

In one instance, the method as defined anywhere herein further comprises reducing the levels of free nucleic acid from dead or dying cells that are encapsulated in droplets. It will be understood that the levels of free nucleic acid from dead or dying cells are reduced as compared to the levels that would be encapsulated if this method step had not been carried out. It will be appreciated by the person skilled in the art that any method for achieving this is within the scope of the present disclosure. In one instance, the levels of free nucleic acid from dead or dying cells are reduced by 10%, 20%, 30%, 40%, 50%, 60%, 70%, 80%,or 90%.

In one instance, the preventing comprises stimulating cells for less than 48 hours prior to encapsulating. In one instance, the reducing comprises stimulating cells in the first aqueous solution comprising cells for less than 48 hours.

In one instance, the preventing comprises selecting live cells prior to encapsulating. In one instance, the reducing comprises selecting live cells for combining with the reagents for amplifying and linking native pairings of amplicons in the aqueous suspension. In one instance, the selecting comprises Fluorescence-activated cell sorting (FACS). In one instance, the selecting comprises bead-based selection of cells. It will be appreciated by the person skilled in the art that the desired cells selected for encapsulating are the live cells and not the dead or dying cells.

In one instance, the preventing comprises sequestering the free nucleic acid from dead or dying cells using magnetic beads. In one instance, the reducing comprises sequestering the free nucleic acid from dead or dying cells from the first aqueous solution using magnetic beads. In one instance, the reducing comprises sequestering the free nucleic acid from dead or dying cells from the aqueous suspension comprising the cells and the reagents. Sequestering the free nucleic acid from dead or dying cells allows removal of this nucleic acid such that the levels of free nucleic acid from dead or dying cells that are encapsulated in droplets are reduced. In one instance, the magnetic beads are oligonucleotide-coated magnetic beads. In one instance, the magnetic beads are poly-dT beads. Magnetised beads with nucleic acid binding agents, in particular RNA binding agents, may be added to the cell suspension prior to encapsulation to 'mop up' free nucleic acid, in particular RNA, present in the surrounding media. The beads may be removed prior to encapsulation using a magnetic field, thereby removing the contaminating nucleic acid.

The present disclosure further provides a method for producing a library of natively-paired amplicons, the method comprising producing encapsulated natively-paired amplicons according to the method as defined above; and lysing the droplets to produce a library of natively-paired amplicons. Commercial kits are available for lysing the droplets (for example, the Micellula kit from EURx). The DNA from the droplets can be purified using a PCR purification kit from Qiagen or by other methods such as using isobutanol (Schutze, T. et al., Anal.Biochem. (2011), 410: 155-157) and diethyl ether (Diehl, F. et al., Nature Methods (2006), 3; 551-559) have been described. A library of natively-paired amplicons produced according to the above method is within the scope of the present disclosure. A library of natively-paired amplicons having the features of a library of natively-paired amplicons produced according to this method is also within the scope of the present disclosure.

The present disclosure further provides a method for producing a library of natively-paired scFv amplicons for screening for antigen binding and/or function, the method comprising producing a library of natively-paired scFv amplicons according to the method as defined above; and producing a further library of natively-paired scFv amplicons, wherein the natively-paired scFv amplicons of the further library have the general formula R1-V1-L-V2-R2, wherein R1 and R2 are the same or different and each comprises a restriction enzyme site, V1 and V2 are the natively-paired heavy and light chain variable domain, wherein when V1 is the light chain variable domain, V2 is the heavy chain variable domain or when V1 is the heavy chain variable domain, V2 is the light chain variable domain, and L is a direct bond or linker. In one instance, the natively-paired scFv amplicons of the further library have the general formula R1-VH-L-VL-R2 i.e. V1 is VH and V2 is VL. In another instance, the natively-paired scFv amplicons of the further library have the general formula R1-VL-L-VH-R2 i.e. V1 is VL and V2 is VH.

In one instance, R1 and R2 as defined anywhere herein are different. L is a linker. Preferably, R1 and R2 are different and L is a linker.

In one instance, the producing a further library of natively-paired amplicons as defined anywhere herein comprises using Nested PCR. Nested PCR uses a second set of primers to amplify the full amplicon, which are different to those used to generate the amplicon. A subsequent run of nested PCR therefore amplifies the final expression-ready amplicon and reduces amplification of alternative PCR products formed due to non-specific primer binding. In one instance, the Nested PCR uses primers that bind to FR1 of V1 and FR4 of the V2. The full scFv amplicon product can therefore be amplified. In one instance, the Nested PCR as defined anywhere herein uses primers that are fused to overhang sequences that comprise a restriction enzyme site. Preferred restriction enzyme sites are Sfi1 and Not1. In one instance, R1 as defined anywhere herein comprises the Sfi1 restriction enzyme site and R2 as defined anywhere herein comprises the Not1 restriction enzyme site. The resulting amplicon forms an expression-ready amplicon that can easily be cloned into expression vectors for any number of expression systems for phage-display panning or direct screening.

A library of natively-paired scFv amplicons for screening for antigen binding and/or function produced by the method as defined above is within the scope of the present disclosure. A library of natively-paired scFv amplicons for screening for antigen binding and/or function having the features of a library of natively-paired scFv amplicons for screening for antigen binding and/or function produced by the method as defined above is also within the scope of the present disclosure.

The disclosure further provides a method for producing a natively-paired scFv library for screening for antigen binding and/or function, the method comprising producing a library of natively-paired scFv amplicons according to the method described herein; and expressing the natively-paired scFv. In one instance, this method comprises expressing the natively-paired scFv as scFv-Fc. In another instance, this method comprises reformatting the natively-paired scFv to IgG. Preferably, this reformatted IgG may be directly screened for binding and function, enriched by phage-display panning, or deep-sequenced using next-generation sequencing for repertoire characterisation. In a further instance, this method comprises expressing the natively-paired scFv as a scFv phage display library.

In one instance, each scFv of the scFv library as defined anywhere herein comprises the heavy and light chain variable domains of a native pairing of a single cell linked together by a linker. The linker must be of a length to allow pairing between the heavy and light chain variable domains. Preferably, the linker as defined anywhere herein is Glycine and/or Serine rich. More preferably the linker as defined anywhere herein is $(Gly_4Ser)_3$ and encoded by the following sequence: ggaggcggcggtagcggcggaggtggctcaggcggtggcggaagt (SEQ ID NO: 168).

The linker as defined anywhere herein has a length of 10-20 amino acids. More preferably, the linker as defined anywhere herein has a length of 13-18 amino acids. Still more preferably, the linker as defined anywhere herein has a length of 15 amino acids. Suitable linkers will be well known to the person skilled in the art.

A natively-paired scFv library for screening for antigen binding and/or function produced by the method as defined above is within the scope of the present disclosure. A natively-paired scFv library for screening for antigen binding and/or function having the features of a natively-paired scFv library for screening for antigen binding and/or function produced by the method as defined above is also within the scope of the present disclosure. Advantageously, the scFv library is expression-ready.

The disclosure further provides a method for identifying an antigen-specific molecule, the method comprising producing a natively-paired scFv library according to the method described herein; interrogating the natively-paired scFv library with an antigen sample; and identifying an antigen-specific molecule.

This technology described herein is exemplified with B-cells and the platform is established for B-cell repertoire capture. Since T-cell receptors can also be converted into scFv format and retain activity (Grégoire et al., European Journal of Immunology (1996), 26 (10): 2410-16), the technology can equally be applied to the capture of the T-cell receptor repertoire.

The present disclosure further provides a scFv library comprising natively-paired recombinant scFv for screening for antibody binding and/or function, wherein each scFv comprises the heavy and light chain variable domains of a native pairing of a single cell linked together as described herein. Instances as hereinbefore described in connection with the methods of the present invention may provide further information regarding features of the libraries. Advantageously, the libraries may be a translatable. The present libraries enable high throughput translation and subsequent screening of the sequences.

The present disclosure further provides a method for identifying an antigen-specific molecule, the method comprising interrogating a natively-paired scFv as defined anywhere herein with an antigen sample; and identifying an antigen-specific molecule. The present disclosure further provides a method for identifying an antigen-specific molecule, the method comprising interrogating a scFv library as defined anywhere herein with an antigen sample; and identifying an antigen-specific molecule.

In one instance, the antigen-specific molecule is an antibody. In one instance, the antibody as defined anywhere herein is a monoclonal antibody.

In one instance, the antigen sample as defined anywhere herein is tumour tissue. In one instance, the antigen sample as defined anywhere herein is whole bacteria. In one instance, the antigen sample as defined anywhere herein is viral particles. In one instance, the antigen sample as defined anywhere herein comprises hemagglutinin (HA) proteins.

It will be appreciated by the person skilled in the art that multiple rounds of interrogating the library can be carried out, preferably with different antigen samples, so as to allow the identification of cross-reactive antigen-specific molecules. In one instance, the antigen-specific molecule is cross reactive.

The present disclosure also provides the use of a natively-paired scFv library as defined anywhere herein for identifying an antigen-specific molecule. The present disclosure also provides the use of a scFv library as defined anywhere herein for identifying an antigen-specific molecule.

In one instance, the use as defined above comprises interrogating the scFv library with an antigen sample and identifying an antigen-specific molecule. All instances as hereinbefore described in connection with the methods of the present invention apply equally to the above defined use.

While the methods defined herein enable isolation of antibodies from human B cells, it can readily be extended to isolate antibodies from any species for which V-gene sequence information is available. This can be particularly useful for expanding the breadth and depth of the hybridoma technology, where low fusion efficiencies (less than 0.02% (Yu, X., et al., J. Immunol. Methods (2008), 336; 142-151)) lead to significant loss of repertoire. The methods defined herein may also be applied for generating monoclonal antibodies from organisms for which myeloma fusion partners are not available. T cell receptor (TCR) repertoires (consisting of paired $\alpha/\beta$ or $\delta/\gamma$ chains) could also be captured in a similar recombinant format and single-chain TCR has been shown to be amenable to selection by phage and yeast display (Li, Y. et al. Nat. Biotechnol. (2005), 23: 349-354; Smith, S. N. et al. Methods Mol. Biol. Clifton NJ (2015), 1319: 95-141).

The methods enable the rapid capture of the native repertoire from millions of primary human B cells into a powerful and sensitive screening platform, with significant implications for therapeutic antibody development, immune repertoire characterization and rational vaccine design. For example, linking the variable domains into a translatable scFv format allows the combination of the strengths of multiple technologies: using the immense screening power of display platforms to mine the full richness of a naturally evolved antibody response.

The present disclosure provides a fast method for lead antibody generation from natural repertoires - a single researcher can rapidly progress from millions of primary B cells to specific monoclonal antibodies within 4 weeks. This could be especially valuable for combating emerging infectious diseases, e.g. an Ebola outbreak.

The libraries of the present disclosure constitute a renewable resource that can be expanded as new donors are added, panned repeatedly against a multitude of targets (including whole bacteria or tumor tissue), or archived indefinitely for future use.

Large-scale efforts that use next generation sequencing to predict antibody function could particularly benefit, such as the recently launched Human Immunome Program (Crowe, J. E. & Koff, W. C. Expert Rev. Vaccines (2015), 14: 1421-1425). This project aims to sequence the expressed antibody repertoires from 1000 individuals and infer vaccine reactivity based on sequencing information alone. An exciting addition to this project could be to use the method outlined here to build pooled display libraries from these individuals, such that one could directly measure the reactivity of the human repertoire to any number of vaccine candidates.

**EXAMPLES**

**Example 1: Primer design**

Primers were designed using custom software written in Perl for maximal coverage of all human Ig sequences (Table 1 and Table 5). Nucleotide sequences for leader, variable and constant regions were downloaded from IMGT (Lefranc et al., Nucleic Acids Research (2009), 37) for the human heavy, lambda and kappa genes (excluding pseudogenes and truncated transcripts) and four sets of primers were designed for each gene family. Outside primers ("out" subscript in the primer names) were designed by calling Primer3 and design primers to span the splice junction of the leader sequence ("out_5" primers) or bind within the first 50 bases of the constant domain ("out_3" primers). For VK_out_3, a manually designed primer was created to span the variable domain-constant domain splice junction. All primers were designed to anneal with a minimal melting temperature of 60°C. Inside primers ("in" subscript in the primer names) were designed by fixing the 5' end of the primer at the start ("in_5") or end ("in_3") of the V coding sequence and extended until the Tm reached 60°C using the OligoTm module from Primer3. FR4 specific primers were also manually extended for increased specificity. Where possible, primers within each set were consolidated to have at most 4 degenerate bases. Where possible, primers within each set were fused to overhangs to enable linker formation (VH_in_3 and VK/L_in_5). Where possible, primers within each set were fused to overhangs to enable restriction digestion by NotI or SfiI. Where possible, primers within each set were barcoded for MiSeq deep sequencing (Table 1).

## Example 2: Recovering natively-paired scFv from cells using Immune Replica technology

*Example 2.1: Encapsulation of primary human B-cells*

This method described below provides a platform to capture the antibody repertoire from pools of primary B-cells into a screenable format while maintaining the cognate heavy and light chain pairing (Figure 1). To achieve this, each B-cell is encapsulated into a water-in-oil droplet containing reagents for RT-PCR amplification of the heavy and light variable domain gene transcripts and their pairing by overlap-extension PCR to generate a scFv amplicon.

B-cells were isolated from healthy human blood samples using the RoboSep Human B-cell Enrichment Kit (StemCell Technologies, 19054RF). Cells were centrifuged at 500xg for 10 minutes and re-suspended in RPMI1640 (Invitrogen, A10491-01), supplemented with insulin-transferrin-selenium (Invitrogen, 41400-045), 10% fetal bovine serum (Invitrogen, 10082-147), 0.5μg/ml megaCD40L (Enzo, ALX-522-110-C010), 33ng/ml IL-21 (internally produced) and penicillin-streptomycin-glutamine (Invitrogen, 10378-016) and grown at 37°C and 5% $CO_2$ for 48 hours. Prior to encapsulation, cells were washed in PBS (3 minutes at 700xg) before re-suspending in hypoosmolar electrofusion buffer (Eppendorf, 940002150) containing 1:1,000 dilution of Anti-Clumping Agent (Invitrogen, 0010057DG) and 0.4mg/ml acetylated BSA (EURx, E4020-01).

Stimulated B-cells have a tendency to aggregate over time and this can cause changes in flow rates, as well as multiple cells being encapsulated together. Use of an anti-clumping excipient and a paramagnetic stir disk were found to keep cells from settling prior to encapsulation.

Acetylated BSA is an amphipathic molecule which can stabilize the water-oil interface and lower the interfacial tension (Dalgleish, Trends in Food Science & Technology (1997), 8 (1): 1-6). Droplet coalescence during the harsh conditions of PCR cycling was optimized. A combination of lower denaturation temperatures and the use of acetylated BSA decreased droplet coalescence and improved droplet stability (Figure 7). Preferably, the denaturation temperatures are within the range of about 85°C and about 95°C. More preferably, the denaturation temperatures are within the range of about 86°C and about 90°C. For instance, the denaturation temperature may be about 88°C. Further, the presence of acetylated BSA may protect enzymes such as reverse transcriptase from denaturation at the interface.

As reagents within the droplets cannot be added or subtracted once the droplet has formed, a reaction mixture was optimized to perform all steps in a single reaction mix. Cells were encapsulated at a 1:1 ratio with 2xRT-PCR master mix. Stock primers were mixed at 100μM in equal amounts to create pools that were added to the RT-PCR mix.

A typical master mix of 300μl was composed of 4.86μl VH-out-F-T7, 4.86μl VL-out-R-T3, 2μl VH-in-R and 2μl VL-in-F (Table 2), 120ul 5x OneStep RT-PCR buffer, 24μl OneStep RT-PCR enzyme mix, 54.1μl Q solution (Qiagen, 210212), 12μl 10mM dNTP (Invitrogen, 18109-017) and 30μl RNaseOUT (Invitrogen, 10777-019). Reagents were left to incubate on ice before centrifuging through a 0.22μm spin filter (Corning, 8169).

The organic phase of the emulsion was made using the Micellula emulsion PCR kit (EURx, 3600-02) using 60% component 1, 20% component 2 and 20% component 3. Reagents were mixed and vortexed for 60 seconds at maximum speed and incubated at room temperature for at least 30 minutes. Organic phase was filtered through a 0.22μm filter prior to encapsulation.

Encapsulation was performed on a 2-reagent droplet generation chip (Dolomite, 3200242) with fluids pumped using an OB1 flow controller (Elvesys). Aqueous liquids of cells and RT-PCR mix were each pumped at 57mbar while the oil liquid was pumped at 101mbar. The resulting emulsion was collected in 6-minute fractions (about 40μl emulsion per fraction) in PCR strip tubes. As a control, an open PCR reaction was made by combining equal volumes of cell and RT-PCR mixes and divided in PCR strip tubes in 40μl aliquots.

It was found that by reducing the relative amount of 'inside' relative to 'outside' primers (Figure 3) by a factor of 8, it was possible to selectively deplete the inside primers during the early PCR cycles and favour production of the full linked product over individual variable domain amplicons (data not shown).

*Example 2.2: Generation of scFv containing natively-paired variable domain genes*

Encapsulated and open reverse-transcription PCR reactions were performed with a reverse transcription step of 30 minutes at 55°C followed by heat-inactivation of RT/activation of Taq polymerase of 2 minutes at 88°C. This was followed by 45 cycles of PCR (88°C for 30 seconds, 62°C for 30 seconds, 72°C for 1 minute) and a final extension step of 10 minutes at 72°C. Excess oil above the droplets was manually removed and the droplets were lysed by adding 5x excess of Buffer PB from the QiaQuick PCR purification kit (Qiagen, 28106) and PCR product was purified according to the manufacturer's instructions. The products were size-selected on 1% agarose to between 600-1200bp using the QiaQuick gel-extraction kit (Qiagen, 28706) and eluted in 40μl EB buffer.

*Example 2.3: Amplification of scFv containing natively-paired variable domain genes*

Nested PCR amplification was performed in 15μl reactions using mixtures of VH-in-F and either VK-in-R or VL-in-R (at 1:50 dilution, Table 2), consisting of 1μl purified RT-PCR product as template, 3μl diluted primer mix, 1.5μl 10x Hifi Platinum PCR buffer, 0.3μl 10mM dNTP, 0.6μl 50mM MgSO$_4$ and 0.06μl Hifi Platinum Taq (Invitrogen, 11304-011). Cycling conditions consisted of an initial denaturation step of 2 minutes at 94°C followed by 45 cycles of PCR (94°C for 15 sec, 61°C for 30 sec, 68°C for 60 sec) and a final extension step of 10 minutes at 68°C. Products were purified using the QiaQuick PCR purification kit and eluted in 40μl EB buffer.

**Example 3: Encapsulation of single hybridoma cells**

In order to demonstrate single-cell encapsulation, two aliquots of 1 million mouse hybridoma cells were stained with red or green fluorescence using CellTracker dyes (Invitrogen, C34552 and C7025) according to the manufacturer's instructions. Stained cells were resuspended in PBS and encapsulated using the conditions described above, substituting the RT-PCR mix with PBS. Droplets were collected in 6-well dishes and imaged at 200x magnification using the Evos FL Auto Cell Imaging System (Invitrogen).

A density of 4 million cells per ml was found to be optimal for obtaining mostly single-cell encapsulation into droplets of approximately 100 μm in diameter (Figure 2). Although a number of empty droplets were generated using this process, these did not contribute to the scFv library as no template cells are present.

**Example 4: Comparison of droplet stability using syringe and pressure pumps**

In order to measure improvements in droplet stability, droplets were generated with mouse hybridoma cells and RT-PCR buffer using two methods. The first method used two syringe pumps (Razel R-99) to deliver aqueous and oil fluids, respectively, to the microfluidic chip. Aqueous fluids were loaded into 1mL syringes and dispensed simultaneously from a single pump at 0.5μl/min, whereas the oil:surfactant solution was loaded into a 3mL syringe and dispensed from a separate pump at 1.5μl/min. The second method was as described above using a pulseless pressure pump. 0.5μl emulsion was transferred to 96 well microtiter plates and imaged at 25x magnification to inspect for coalescence and droplet homogeneity. The emulsions were then subjected to 45 cycles of RT-PCR (as described above) and imaged once again.

Optimized PCR conditions and excipients contributed to increased stability, as did generating monodisperse droplets (Figure 7).

**Example 5: Validation of native chain pairing of scFv by Immune Replica technology**

To test the optimization at achieving single-cell encapsulation and droplet stability during RT-PCR, a mixture of primary human and mouse B-cells were used and primer sets were designed to amplify and link the C$_{H1}$ and C$_κ$ domains.

In more detail, primary human B-cells from healthy donors were processed and stimulated as described above. Primary mouse B-cells were isolated from splenocytes using the Mouse B-cell Isolation Kit (StemCell Technologies, 19854) according to the manufacturer's instructions. These cells were stimulated in identical conditions as human B-cells, substituting megaCD40L with mouse CD40L (Enzo, ALX-522-120-C010) and mouse IL21 (internally produced). 48-hour stimulated cells were combined in a 1:1 ratio and 10,000 cells were encapsulated as described above. A parallel "open" reaction was performed by combining 10,000 cells directly in RT-PCR mix without encapsulation. ScFv-like amplicons were generated using RT-PCR and nested PCR as described above, with primer sets designed to amplify and pair the C$_{H1}$ and C$_κ$ domains.

Using the constant instead of the variable domains greatly reduced the complexity of expected outputs to just 4 possibilities: two amplicons with paired fragments (hC$_{H1}$-hC$_κ$ and mC$_{H1}$-mC$_κ$) and two amplicons with scrambled fragments (hC$_{H1}$-mC$_κ$ and mC$_{H1}$-hC$_κ$) which were easily detectable by nested PCR with specific primers and confirmed by Sanger sequencing. As expected, all possible products were identified using an open reaction but strikingly the Immune Replica technology only generated correctly paired amplicons (Figure 4). This provides evidence that chain pairing is maintained within droplets.

## Example 6: Isolation of antigen-specific scFv from primary human B-cells

### Example 6.1: Determination of serum titers to common antigens

Serum from two healthy donors (642 and 432) was isolated by centrifugation of whole blood at 500xg for 10 minutes, then diluted in ELISA blocking buffer (3% nonfat milk - Bio-Rad, 106404XTU + 0.1% Tween-20 - BDH,BDH4210 in PBS). C. albicans mannan and Influenza hemagglutinin (South Dakota) antigens were produced in-house and coated on 96-well High Binding plates (Corning, 3690) at 4 $\mu$g/ml and incubated overnight at 4°C. Plates were blocked in ELISA blocking buffer for 2 hours before being washed 3 times with ELISA washing buffer (PBS + 0.05% Tween-20) and incubated with serial dilutions of the sera for 1 hour. Plates were washed 3 times and bound IgG was detected with a 1:10,000 dilution of anti-human Fc-gamma-HRP (Jackson labs, 109-035-098), with TMB development over 5 minutes (KPL, 52-00-04). The reaction was stopped by adding an equal volume of 1M hydrochloric acid before colorimetric analysis was performed by measuring absorbance at 450nm.

Both donors had moderate serum titers against two common therapeutic targets: Influenza hemagglutinin (H1, South-Dakota variant) and *C. albicans* mannan (Figure 8).

### Example 6.2: Isolation of antigen-specific scFv

In order to validate the Immune Replica technology, a head-to-head comparison of libraries generated from primary human B-cells with and without single-cell encapsulation was conducted.

For each of the two healthy donors (642 and 432), approximately 10,000 primary B-cells were encapsulated ("em") with the full set of primers for human variable gene amplification and chain pairing as described above. In parallel, reference libraries were also generated for each donor using 10,000 primary B-cells that were not encapsulated (open ("op") reaction), where variable gene pairings are expected to be scrambled.

Following RT-PCR and nested PCR amplification, scFv amplicon bands were subcloned into a scFv-Fc expression vector with NotI/SfiI (New England Biolabs cat no R0189S and R0123S) for high-throughput screening by ELISA (Figure 5).

Transformants from the 4 libraries (642-em, 642-op, 432-em and 432-op) were plated on Qtrays containing 2xYT agar+ 100 $\mu$g/ml carbenicillin + 2% glucose (Teknova, Y6260) and 1,408 colonies were picked for each into 384-well plates containing 60ul LB + 100 $\mu$g/ml carbenicillin (Invitrogen, 10177-012) + 2% glucose (Teknova, G0535) and grown overnight at 37°C. 5 $\mu$l of the overnight culture was used to inoculate 384 deep-well plates containing 250 $\mu$l reconstituted MagicMedia (Invitrogen, K6803) + 100 $\mu$g/ml carbenicillin and grown over 3 days at 25°C. After 3 days the cultures were treated with 1:10 dilution of PopCulture reagent (Novagen, 71092) + 1:10,000 dilution of DNAse I (Invitrogen, 18047-019) and debris was cleared by centrifugation at 4000xg for 15 minutes. Antigen binding was performed by ELISA, as described above, except that the blocking step used 3% BSA (Sigma, A7030) + 0.05% Tween-20 (BDH, BDH4210) in PBS. Antibodies were deemed specific to the antigen where the signal was greater than the mean background signal plus 20-times the background standard deviation. Hits were verified by re-expressing the antibody from glycerol stocks and repeating the ELISA.

Of the 5,632 colonies that were screened for binding to immobilized antigen, 5 binders to hemagglutinin were identified and 1 binder to mannan (Table 3). The majority of hits (5/6) came from the droplet libraries, suggesting that by preserving native chain pairing the identification of clones that may be lost during construction of a combinatorial library is facilitated.

### Example 6.3: Deep sequencing analysis of V/J germline diversity

In parallel, the repertoire of the encapsulated and open libraries was characterized by paired-end Illumina MiSeq deep sequencing.

As scFv amplicons are too large for MiSeq deep sequencing, separate $V_H$ and $V_{K/L}$ fragments were amplified using specific primer sets (Table 1). Nested PCR amplification was performed in 15 $\mu$l reactions using mixtures of iVH-in-F/iVH-in-R, iVK-in-F/iVK-in-R and iVL-in-F/iVL-in-R (at 1:50 dilution, Table 2), consisting of 1 $\mu$l template, 3 $\mu$l 1:50 dilution of primer mix (VH-in-F + VL-in-R), 1.5 $\mu$l 10x Hifi Platinum PCR buffer, 0.3 $\mu$l 10mM dNTP, 0.6 $\mu$l 50mM $MgSO_4$ and 0.06 $\mu$l Hifi Platinum Taq (Invitrogen, 11304-011). Cycling conditions consisted of an initial denaturation step of 2 minutes at 94°C followed by 45 cycles of PCR (94°C for 15 sec, 61°C for 30 sec, 68°C for 60 sec) and a final extension step of 10 minutes at 68°C. Products were submitted for MiSeq 2x250bp paired-end sequencing (SeqMatic). Raw reads were quality filtered based on the reported quality score in the FASTQ files. Unique reads were mapped to IMGT V and J gene germline sequences to determine any biases that may have been introduced as a result of amplification within droplets.

All gene families that were detected in the open library were also identified within the encapsulated one at very similar proportions, suggesting that droplet amplification does not bias the repertoire (Figure 6).

**Example 7: Process optimization for increasing the throughput of Immune Replica libraries**

In order to increase the throughput of the Immune Replica technology to $10^6$ cells, a less viscous oil carrier (fluorinated oil) was used to enable the use of greater flow rates. $10^6$ cells counted by ViCell were successfully encapsulated within 30-40 minutes.

RT-PCR components, cell encapsulation buffers and microfluidic flow rates were further optimized to generate droplets from $10^6$ B cells that had improved stability during RT-PCR and were homogenous in size.

**Example 8: Recombinant human B cell repertoires enable screening for rare, specific and natively-paired antibodies**

The method described below shows the capture of two million primary B cells into natively-paired expressible libraries that can be directly enriched and screened for function, while still maintaining the ability to profile the paired repertoire by next-generation sequencing (Figure 1). This is achieved by encapsulating B cells into picoliter-sized droplets (approximately 200 pL in volume), in which their cognate V genes are fused in-frame to form an scFv cassette. Glass microfluidic chips were used with pressure pumps to reliably generate evenly sized droplets at high rates, such that one million B cells could be encapsulated within 40 minutes.

The power of this approach is demonstrated by constructing natively-paired phage-display libraries from the peripheral blood cells of two healthy donors, which allowed selection to be driven towards antibodies cross-reactive to multiple influenza hemagglutinin (HA) subtypes.

Progression from whole blood isolation to 18 unique anti-HA monoclonal antibodies was achieved within four weeks. Six of these antibodies were cross-reactive to multiple HA subtypes, including one that showed cross-reactivity to 10 different subtypes from influenza A (Group 1 and 2) and B lineages. The vast majority of these antibody sequences were not detected by next-generation sequencing of the paired repertoire, illustrating how this method can isolate extremely rare leads not likely found by existing technologies.

*Example 8.1 : Capture of the paired immunoglobulin repertoire of primary human B cells into an expressible format using the optimized process for increased throughput*

To capture the paired immunoglobulin repertoire into an expressible format, primer sets for multiplex amplification of all known human V and J genes were computationally designed from IMGT consensus sequences as described in Example 1. In total, 92 primers were designed to amplify the 542 functional human V and J alleles with the appropriate overhangs for scFv generation (Table 5).

Total B cells from healthy donors were isolated and stimulated as described in the examples above. For memory B cell isolation, the Human Switched Memory B Cell Isolation Kit (Miltenyi Biotec) is further used.

Two million B cells were separately isolated from the blood of two healthy donors: total B cells from Donor 1 and $IgG^+/IgA^+$ switched memory B cells from Donor 2.

For each donor, the cells were washed in PBS (3 minutes at 700g) and split into two halves.

One million cells were encapsulated with the optimized RT-PCR mix to generate natively-paired amplicon libraries ("emulsion library"). Specifically, one million cells were re-suspended in encapsulation buffer: hypo-osmolar electrofusion buffer (Eppendorf, 940002001) containing 1:1,000 dilution of Anti-Clumping Agent (Invitrogen, 01-0057AE) and 16% OptiPrep Density Gradient medium (Sigma, D1556).

Cells were encapsulated at a 1:1 ratio with 2xRT-PCR master mix. The primers within each set were mixed in equal amounts and optimised concentrations of each set were added to the RT-PCR mix. A typical 2x master mix was composed of 139nM VH-out-F, 416nM VL-out-R, 39nM VH-in-R and 13nM VL-in-F (Table 3), 2x One Tube RT-PCR reaction buffer (Roche cat no 11855476001), 4% Titan One Tube RT-PCR enzyme mix (Roche, 11855476001), 18.2% Q solution (Qiagen, 210212), 0.4mM dNTP (Invitrogen, 18427013), 10mM DTT (Roche, 11855476001) and 120 units RNaseOUT (Invitrogen, 10777019).

Encapsulation was performed on a 2-reagent droplet generation fluorophilic chip (Dolomite, 3200510) with fluids pumped using an OB1 flow controller (Elveflow, MKII). Aqueous liquids of cells and RT-PCR mix were each pumped at 30mbar while HFE-7500 fluorinated oil + 2% w/v 008-fluoro-surfactant (RAN Biotechnologies, 008-FLUOROSURFACTANT-HFE7500) was pumped at 67mbar, with pressures fine-tuned to obtain a 1:1 mix of aqueous fluids. The resulting emulsion was collected in fractions (about 40μl emulsion per fraction) in PCR strip tubes and overlaid with mineral oil. Excess fluorinated oil was removed to maintain the overall volume at 100μL.

It was found that, using this method, it was possible to encapsulate one million B cells within 40 minutes. This method allows reliable generation of evenly sized droplets at high rates.

The remaining million cells were used to build a combinatorial scFv library ("combinatorial library"). Specifically, one million cells were processed for total RNA using the RNEasy RNA isolation kit (Qiagen) according to the manufacturer's instructions. 250ng total RNA was used for RT-PCR using the same master mix as with emulsions, except that the $V_H$ and $V_L$ sequences were amplified separately and then paired by overlap extension PCR (using the same primer sets).

*Example 8.2: Amplification of scFv containing natively-paired V-genes*

Encapsulated and combinatorial libraries were created by reverse transcription for 30 minutes at 50°C followed by heat-inactivation of RT/activation of Taq polymerase of 2 minutes at 88°C. This was followed by 45 (emulsion) or 35 (combinatorial) cycles of PCR (88°C for 10 seconds, 62°C for 30 seconds, 68°C for 45 seconds) and a final extension step of 7 minutes at 68°C. Excess oil below the droplets was manually removed and the droplets chemically coalesced using an equal volume of Pico-Break 1 (Dolomite). Amplified DNA was size-selected on 2% agarose using the QIAquick gel-extraction kit (Qiagen).

Though it has been reported by several groups that cell-based RT-PCR is not feasible in volumes of less than 5 nL (DeKosky, B.J. et al., Nat. Biotechnol. (2013), 31: 166-169; DeKosky, B.J. et al., Nat. Med. (2015), 21: 86-91; White, A. K. et al., Proc. Natl. Acad. (2011), 108: 13999-14004; Eastburn, D. J. et al., PloS ONE (2013), 8: e62961), it was found that this method is able to successfully amplify Ig transcripts directly from cells in droplets of approximately 200 pL in volume.

Both cases resulted in a linked product consisting of (from 5' to 3') part of the $V_H$ leader sequence, $V_H$, $(Gly_4-Ser)_3$ linker, $V_L$, N-terminus of $C_L$. This product was then used as template for nested PCR with $V_H$ FR1 and $V_L$ FR4 specific primer sets to generate full-length scFv amplicon libraries (Figure 13A).

To obtain an in-depth assessment of the captured repertoire, the nested PCR primers contained barcoded overhangs that enable next-generation sequencing on the Illumina MiSeq (Figure 13A).

Nested PCR amplification consisted of 25% purified RT-PCR product, 100nM VH-in-F and VL-in-R primer pools (Table 5), 1x Hifi Platinum PCR buffer, 0.15mM dNTP, 1.5mM $MgSO_4$ and 0.6 units Hifi Platinum Taq (Invitrogen). Cycling conditions consisted of an initial denaturation step of 2 minutes at 94°C followed by 50 cycles of PCR (94°C for 30 sec, 55°C for 30 sec, 68°C for 60 sec) and a final extension step of 10 minutes at 68°C. Products were again size-selected as above.

A final scale-up PCR was performed using common forward (Illu scaleup_F) and barcoded reverse primers (Illu_R_N50X) to enable library construction and Illumina sequencing (Table 6). We used the Q5 polymerase (NEB) according to manufacturer's instructions with the following thermocycling program: 98°C for 2 minutes, 12-20 cycles of 98°C for 10 seconds and 72°C for 30 seconds, 72°C for 2 minutes.

*Example 8.3: Next generation sequencing and bioinformatics analysis*

Each barcoded library was size-selected to 850bp, combined in equal amounts and subjected to 2×300bp MiSeq sequencing using a custom priming approach (SeqMatic). The custom priming strategy was designed to obtain paired 300bp reads of the 3' ends of $V_H$ and $V_L$, consisting of FR4, CDR3 and FR3 (Figure 14). The R1 and R2 primers (Table 6) were used to generate $V_L$ and $V_H$ sequences, respectively, whereas the standard Illumina P5 primer was used for the index read. Whereas the $V_L$ read was obtained using a priming site introduced at the 3' end of the construct, the $V_H$ read required an internal primer annealing to the $(G_4S)_3$ linker sequence (Table 6).

Following demultiplexing, raw Fastq reads were quality-filtered using FastQC, paired by the Illumina Fastq ID, aligned to IMGT V and J genes and annotated according to Kabat definition (Lloyd, C. et al. Protein Eng. Des. Sel. PEDS (2009) 22: 159-168) to extract CDR3 sequences. Subsequently, CDRH3 and CDRL3 sequences were concatenated and clustered where the amino acid identity was greater than 92%.

Unique CDRH3 and CDRL3 sequences were counted and the numbers of unique VL sequences pairing with each unique VH were calculated as a measure of pairing efficiency. For CDRH3 sequences paired with multiple CDRL3, the top-pair weight is determined as the ratio of counts between the most abundant CDRL3 and all CDRL3 sequences (DeKosky, B.J. et al., Nat. Med. (2015), 21: 86-91). A total of 266,344 and 2,666,926 unique CDRH3:CDRL3 clusters were recovered for the two emulsion and combinatorial libraries, respectively (Table 7).

The clustering parameters were validated using the error-corrected asymptotic Chao richness estimator (Chiu, C.-H. & Chao, A. PeerJ (2016), 4: e1634) and it was found that the computed diversity of the amplicon library adjusted for sequencing artifacts is very close to the observed number of clusters. This provides evidence that the clustering parameters reliably corrected for sequencing errors while minimising the loss of truly unique sequences.

*Example 8.4: Validation of native chain pairing of antibodies*

*Example 8.4.1: Validation using a mouse and human B-cell mixture*

To validate this approach at achieving single-cell encapsulation and cognate chain pairing, primary human and mouse B cells were isolated and stimulated as described in Example 5. Equal amounts of primary stimulated mouse and human B cells were mixed and 10,000 cells from this mixture were encapsulated as described in Example 8.1.

A parallel "combinatorial" reaction was performed by combining 10,000 cells directly in RT-PCR mix without encapsulation. RT-PCR and nested PCR conditions were as described in Example 8.2 with primer sets designed to amplify and pair the $C_{H1}$ and $C_K$ domains (Table 3).

As expected, the combinatorial format produced all possible products but, strikingly, only natively-paired amplicons were generated with encapsulation (Figure 4b).

*Example 8.4.2: Validation of native chain pairing of scFv by spike-in experiment*

One million total B cells isolated from the blood of a healthy donor were mixed with 10,000 IM-9 cells (1%) before being encapsulated with the optimised RT-PCR mix to generate a natively-paired amplicon library, consisting of (from 5' to 3') part of the $V_H$ leader sequence, $V_H$, $(Gly_4$-Ser$)_3$ linker, $V_L$, N-terminus of $C_L$. This product was then used as template for nested PCR with $V_H$ FR1 and $V_L$ FR4 specific primer sets to generate a full-length scFv amplicon library (Figure 13A).

As a further validation of correct chain pairing, a primer specific to the IM-9 CDRH3 sequence (RRGVTDIDPFDI; IM9-CDRH3-Fwd) was used with a generic reverse primer (R1, Table 6) to amplify all $V_L$ sequences that paired with the IM-9 heavy chain. The resulting amplicon was cloned and analysed by Sanger sequencing, which showed correct pairing with the known IM-9 $V_L$ (QHYNRPWT) in 97/101 colonies (96% pairing accuracy). This confirms that even with an overwhelming abundance of competing B cells; the present method maintains correct chain pairing.

*Example 8.4.3: Determination of the number of unique CDRL3 sequences pairing with each CDRH3 sequence*

As a yet further validation that the Immune Replica system preserves chain pairing, the number of unique CDRL3 sequences that paired with each CDRH3 sequence was determined.

As expected, the combinatorial library displayed promiscuous pairing, with each CDRH3 sequence paired with a median of 5-9 unique CDRL3 sequences (Figure 13B, Table 7). Given that the sequencing depth ($10^6$) vastly under-samples the theoretical sequence diversity of the combinatorial libraries ($10^{12}$), the true rate of combinatorial pairing would likely be considerably higher. This was in stark contrast to the emulsion libraries, where a median of 1:1 CDRH3/CDRL3 pairing with narrow distribution was observed. In cases where multiple pairings were detected, top-pair analysis determined a 95%-98% accuracy in $V_H$-$V_L$ pairing (Figure 13C). The top-pair method has been used to validate cognate chain pairing in a previously published method that generates amplicons only suitable for sequencing, but not for screening (DeKosky, B.J. et al., Nat. Med. (2015), 21: 86-91). However, using similar sequencing depth and starting cell numbers the pairing efficiency was found to be significantly better with the present method ($p < 0.007$, Figure 15 and Table 8).

*Example 8. 5: Imaging of single encapsulated a cells*

B cells were stained using CellTracker Red CMTPX or CellTracker Green CMFDA dyes (Life Technologies) according to the manufacturer's instructions. Stained cells were re-suspended in PBS and encapsulated using the conditions described above, substituting the RT-PCR mix with PBS. Cell lysis was imaged in two ways: (1) stained cells were re-suspended in encapsulation buffer, encapsulated with RT-PCR mix and heated to 50°C for 5 minutes; (2) unstained cells were encapsulated with RT-PCR mix containing 2x SYBR-Green (Invitrogen) and heated to 50°C for 5 minutes. Droplets were collected in $\mu$-Slide$^{0.1}$ channel slides (Ibidi) and imaged at 200x magnification using the Evos FL Auto Cell Imaging System (Invitrogen).

Robust cell lysis caused by addition of RT-PCR buffer and incubation at 50°C, was observed by Trypan Blue staining (Figure 10), and detection of release of cytosolic dyes and nuclear material by SYBR-Green (Figure 11).

*Example 8.6: Phage display library construction and enrichment for anti-hemagglutinin antibodies*

As proof of principle, the scFv libraries were used to isolate antibodies against influenza hemagglutinin, an antigen to which humans are commonly exposed.

*Example 8.6.1: Isolation of antibodies using the scFv libraries*

The emulsion and combinatorial libraries were bulk subcloned into a phagemid vector (Vaughan, T. J. et al., Nat. Biotechnol. (1996), 14: 309-314) to construct phage-display libraries of over $1 \times 10^8$ transformants.

Specifically, the emulsion and combinatorial amplicon libraries were subcloned into a phagemid vector (pCANTAB6) using Not1/Sfi1 restriction enzymes (NEB) and phage display libraries were generated as described in Vaughan, T. J. et al., Nat. Biotechnol. (1996), 14: 309-314. 96 colonies from each of the 4 libraries were cultured to mid-log phase and infected with M13-K07 (Invitrogen) to initiate overnight monoclonal phage production. Antibody display was determined by ELISA. 1$\mu$g/ml anti-myc antibody (Invitrogen) was immobilised overnight on 96 well MAXISORP plates (Nunc) and blocked for 2 hours with 3% BSA (Sigma) and 0.05% Tween-20 (BDH). Following washing with PBST (PBS pH7.2 (Invitrogen) + 0.05% Tween-20), diluted phage supernatant was bound and detected using an anti-M13-HRP antibody (1:5000, GE Healthcare) and visualised with TMB (KPL). Monoclonal phage ELISA against the myc tag fused to the scFv indicated that the libraries mostly displayed scFv well, with positive display seen for 90-99% of clones (Table 9).

Recombinant hemagglutinin proteins were expressed and purified as described in Benjamin, E. et al.,

J. Virol. (2014), 88: 6743-6750. HA proteins used are as follows: H1 CA/09, A/California/07/2009 H1N1; H1 SD/07, A/South Dakota/06/2007 H1N1; H2 MO/06, A/Swine/Missouri/2006 H2N3; H5 VN/04, A/Vietnam/1194/2004 H5N1; H6 HK/97, A/teal/Hong Kong/W312/97 H6N1; H9 HK/97, A/chicken/Hong Kong/G9/97 H9N2; H3 PE/09, A/Perth/16/2009 H3N2; H7 NL/03, A/Netherlands/219/2003 H7N7; B FL/06, B/Florida/04/2006 Yamagata lineage; B BR/08, B/Brisbane/60/2008 Victoria lineage).

The four libraries were subjected to two rounds of enrichment using used 75nM biotinylated hemagglutinin H1 (A/California/07/2009 H1N1) as described in Vaughan, T. J. et al., Nat. Biotechnol. (1996), 14: 309-314.

Amplified phage outputs were profiled by polyclonal ELISA, using immobilised NeutrAvidin (Thermo Fisher Scientific) to capture specific biotinylated antigen prior to incubation with phage. Polyclonal phage ELISA confirmed robust enrichment for specific hemagglutinin H1 binders regardless of the B cell source (Figures 16A and 16B, Figure 17). Of note, while the combinatorial libraries showed an overall stronger specific enrichment, monoclonal sequencing of the enriched clones revealed a strong bias (90%) for IGHV1-69 germline sequences as compared to the corresponding emulsion library (2.9%, Figure 18).

Since it has previously been shown that IGHV1-69 containing antibodies can contact group 1 hemagglutinin subtypes through heavy chain interactions alone (Pappas, L. et al., Nature (2014), 516: 418-422), it is suggested that enrichment of combinatorial libraries was driven by selecting for $V_L$ partners to IGHV1-69 that expressed or folded well in bacteria. This highlights a key bias with combinatorial libraries.

*Example 8.6.2: Specific enrichment for cross-reactive antibodies*

To specifically enrich for cross-reactive antibodies, the first round output was panned from the emulsion libraries on a non-circulating group 1 subtype, influenza A hemagglutinin H5 (75nM, A/Vietnam/1203/2004).

Enriched libraries were bulk subcloned into an scFv-Fc expression vector (Xiao, X. et al. PLoS ONE (2015), 10: e0140691) using Not1/Sfi1 restriction enzymes (NEB) and transformed into chemically competent Top10 cells (Invitrogen). Single clones were grown overnight in LB containing 100µg/ml carbenicillin (Invitrogen) and 2% Glucose (TekNova) before being diluted 1:500 in reconstituted MagicMedia (Invitrogen) containing 100µg/ml carbenicillin (Invitrogen). Cells were induced over 72 hours at 25°C and pelleted by centrifugation. Diluted supernatants were used to determine antigen reactivity by ELISA as described above, using an anti-Fc-gamma-HRP secondary antibody (Jackson ImmunoResearch). Following sequencing, unique clones were expressed in HEK-293 Freestyle cells for 6 days and supernatants were used to confirm binding by ELISA.

Of the 5,632 clones screened, 320 clones showed specific binding to H1, consisting of 18 unique sequences. This included six unique antibodies that showed cross-reactivity to both antigens used in the panning (Figure 16C). Surprisingly, one of these antibodies (IGHV1-18/IGLV1-44) displayed specific binding to all 10 HA subtypes tested, including subtypes from the A (Group 1 and 2) and both lineages of influenza B (Yamagata and Victoria), with relatively similar $EC_{50}$ values ranging between 16nM to 51nM (Figure 16D). Such universal anti-influenza antibodies are thought to be extremely rare, having long been sought out, yet only identified once by combinatorial phage-display (Dreyfus, C. et al. Science (2012), 337: 1343-1348). Whereas the existence of such an antibody within the healthy donor sample used was unexpected, the ability to isolate it through deep mining of the repertoire illustrates the power of the present method.

*Example 8.6.3: Relative frequency of hits ascertained by CDRH3:CDRL3 pairing*

To ascertain the relative frequency of the hits within the captured B cell repertoire, their respective CDRH3:CDRL3 pairs were searched within the next generation sequencing dataset, allowing for up to 4 amino acid mismatches to account for possible sequencing-induced mutations. Only one of the 18 antigen-specific sequences was observed among the 266,344 unique paired sequence clusters, implying that the remaining hits were too rare to be detected by next generation sequencing. This sequence (0089EA-C02) accounted for 2 out of 4,956,249 mapped reads (Table 7). Following selection, it was found this clone repeated in 32 out of the 5,632 clones screened, an enrichment of 14,000 fold.

It will be appreciated by the person skilled in the art that other platforms for displaying native human antibodies are equally applicable, and the inventors have explicitly contemplated such alternative display systems. For example, yeast display systems might allow the identification of further antigen-specific sequences which might have existed in the repertoire, but were not selected because of differences in expression and folding of human antibodies in bacteria.

As this platform depends on successful PCR from gene-specific primers, it is possible that antibody genes mutated within the primer binding sites may be excluded from the resulting library. Ancestral antibodies of equal activity yet having fewer mutations (Macagno, A. et al. J. Virol. (2010), 84: 1005-1013) could still be captured.

Nevertheless, this particular set of leads could not have been predicted from sequencing information alone. To the extent that the scarcity of these leads determined by next generation sequencing represents that within the original B cell pool, this is suggestive that these leads could not be found through standard methods of culturing and screening individual B cells.

### Example 9: Cell lysis prior to encapsulation can contaminate droplets with RNA

It was thought that encapsulation of free RNA coming from dead or dying cells before they have entrained into droplets could interfere with the isolation of natively-paired libraries. In particular, the RNA encoding VH or VL domains might contaminate droplets and lead to non-natively paired products. In order to determine whether cell lysis prior to encapsulation can contaminate droplets with RNA, 48h stimulated cells were incubated for the same duration (30 minutes) as a typical encapsulation and then bulk RT-PCR of VH domains was performed from either the supernatant or the cell pellet (positive control). A water control was included as a negative control.

Results indicate that a significant amount of RNA is released from the cells (Figure 12).

Methods to mitigate this issue include reduced stimulation time, more stringent selection of live cells (e.g. FACS, bead-based), and sequestering RNA using oligo-coated magnetic beads.

### Example 10: Theoretical single-cell encapsulation percentage

Theoretically, the number of cells encapsulated in a single droplet follows a Poisson distribution with $\lambda = 0.1$, given that the ratio between cells and droplets is 1:10.

$$p(k) = \frac{\lambda^k e^{-\lambda}}{k!} \text{ where } \lambda = 0.1$$

Single-cell encapsulation percentage is defined as the percentage of the droplets with single cell out of droplets with $\geq 1$ cell, therefore, the probability could be calculated as:

$$\frac{p(k=1)}{1-p(k=0)} = 95.08\%$$

Titration of the cell suspension achieves approximately 1 cell for every 10 droplets which, based on Poisson statistics, results in single-cell encapsulation with >95% probability (Figure 9).

### Claims

1. A method for producing encapsulated natively-paired scFv amplicons, the method comprising:

   a. encapsulating single B-cells in droplets, wherein the droplets further contain reagents for amplifying and linking native pairings of heavy and light chain variable domain amplicons from single encapsulated B-cells;
   b. lysing the single encapsulated B-cells; and
   c. generating the encapsulated natively-paired scFv amplicons, wherein each scFv amplicon comprises a native pairing of heavy and light chain variable domain amplicons linked together by a linker, wherein the linker has a length of 10-20 amino acids.

2. The method according to Claim 1, wherein the reagents comprise primers designed to human Ig sequences.

3. The method according to Claim 2, wherein the reagents comprise a primer pool comprising primers having the sequences of SEQ ID NOs:1-94 or having the sequences of SEQ ID NOs: 1-3, 169, 76-79, 170, 171, 82-91, 172, 93, 94, 35, 37, 173, 174, 39, 40, 41, 175, 43, 44-46, 176, 48-54, 56-60, 177, 62, 178-184, 7-10, 185, 12, 13, 15-19, 186-189, 20-22, 190, 24, 191, 26-34, 63, 192, 65, 66 and 193.

4. The method according to any one of Claims 1 to 3, wherein generating the encapsulated amplicons comprises initially forming heavy and light chain variable domain amplicons from native heavy and light chain variable domain sequences and the reagents comprise a primer pool comprising

   a. first and second heavy chain variable domain primers; and
   b. first and second light chain variable domain primers,

   wherein the first heavy chain variable domain primer and the first light chain variable domain primer interact to join the heavy and light chain variable domain amplicons.

5. The method according to Claim 4, wherein the primer pool comprises a lower concentration of the first primers than the second primers.

6. The method according to Claim 4 or Claim 5, wherein the first heavy chain variable domain primer is fused to a first overhang sequence and the first light chain variable domain primer is fused to a second overhang sequence, wherein the overhang sequences interact to join the heavy and light chain variable domain amplicons.

7. The method according to Claim 6, wherein the first and second overhang sequences are at least partially complementary.

8. The method according to any one of Claims 4 to 7, wherein

    a. the first heavy chain variable domain primer is the reverse primer which binds inside the heavy chain variable domain of the native sequence/amplicon, and the second heavy chain variable domain primer is the forward primer which binds outside the heavy chain variable domain of the native sequence/amplicon; and
    b. the first light chain variable domain primer is the forward primer which binds inside the light chain variable domain of the native sequence/amplicon, and the second light chain variable domain primer is the reverse primer which binds outside the light chain variable domain of the native sequence/amplicon.

**9.** The method according to any one of Claims 1-8, wherein the reagents comprise Titan (Roche cat no 11855476001).

**10.** The method according to any one of Claims 1 to 9, wherein the encapsulating comprises using microfluidics.

**11.** The method according to any one of Claims 1 to 10, wherein the encapsulating comprises combining an aqueous suspension with an oil to form an emulsion comprising the encapsulated single cells in droplets, wherein the aqueous suspension comprises the cells and the reagents for amplifying and linking native pairings of amplicons.

**12.** The method according to any one of Claims 1 to 11, wherein generating the encapsulated amplicons comprises the use of RT-PCR.

**13.** The method according to any one of Claims 1 to 12, the method further comprising preventing at least some free nucleic acid from dead or dying cells from being encapsulated in droplets.

**14.** The method according to Claim 13, wherein the preventing comprises stimulating cells for less than 48 hours prior to encapsulating, wherein the preventing comprises selecting live cells prior to encapsulating, or sequestering the nucleic acid using oligonucleotide-coated magnetic beads.

**15.** A method for producing a library of natively-paired amplicons for screening for antigen binding and/or function, the method comprising

    a. producing encapsulated natively-paired amplicons according to the method of any one of Claims 1-14; and
    b. lysing the droplets to produce a library of natively-paired amplicons, wherein each of the scFv amplicons encode a scFv comprising a heavy chain variable domain and a light chain variable domain linked together by a linker, wherein the linker has a length of 10-20 amino acids.

**Patentansprüche**

**1.** Verfahren zum Herstellen von eingekapselten, nativ gepaarten scFv-Amplikons, wobei das Verfahren umfasst:

    a. Einkapseln einzelner B-Zellen in Tröpfchen, wobei die Tröpfchen zudem Reagenzien zum Amplifizieren und Verknüpfen nativer Paarungen von variablen Schwere- und Leichtekettendomänen-Amplikons aus einzelnen eingekapselten B-Zellen enthalten;
    b. Lysieren der einzelnen eingekapselten B-Zellen; und
    c. Erzeugen der eingekapselten nativ gepaarten scFv-Amplikons, wobei jedes scFv-Amplikon eine native Paarung von variablen Schwere- und Leichtekettendomänen-Amplikons umfasst, die durch einen Linker miteinander verknüpft sind, wobei der Linker eine Länge von 10 bis 20 Aminosäuren aufweist.

**2.** Verfahren nach Anspruch 1, wobei die Reagenzien Primer umfassen, die für menschliche Ig-Sequenzen ausgelegt sind.

**3.** Verfahren nach Anspruch 2, wobei die Reagenzien einen Primer-Pool umfassen, umfassend Primer mit den Sequenzen von SEQ ID NO: 1-94 oder mit den Sequenzen von SEQ ID NO: 1-3, 169, 76-79, 170, 171, 82-91, 172, 93, 94, 35, 37, 173, 174, 39, 40, 41, 175, 43, 44-46, 176, 48-54, 56-60, 177, 62, 178-184, 7-10, 185, 12, 13, 15-19, 186-189, 20-22, 190, 24, 191, 26-34, 63, 192, 65, 66 und 193.

**4.** Verfahren nach einem der Ansprüche 1 bis 3, wobei das Erzeugen der eingekapselten Amplikons das anfängliche Bilden von variablen Schwere- und Leichtekettendomänen-Amplikons aus nativen variablen Schwere- und Leichtekettendomänen-Sequenzen umfasst und die Reagenzien einen Primer-Pool umfassen, umfassend

    a. erste und zweite Primer für die variable Schwerekettendomäne und
    b. erste und zweite Primer für die variable Leichtekettendomäne,

wobei der erste Primer für die variable Schwerekettendomäne und der erste Primer für die variable Leichtekettendomäne interagieren, so dass die variablen Schwere- und Leichtekettendomänen-Amplikons miteinander verbunden werden.

5. Verfahren nach Anspruch 4, wobei der Primer-Pool eine geringere Konzentration der ersten Primer als der zweiten Primer umfasst.

6. Verfahren nach Anspruch 4 oder Anspruch 5, wobei der erste Primer für die variable Schwerekettendomäne an eine erste Überhangsequenz fusioniert ist und der erste Primer für die variable Leichtekettendomäne an eine zweite Überhangsequenz fusioniert ist, wobei die Überhangsequenzen interagieren, so dass die variablen Schwere- und Leichtekettendomänen-Amplikons miteinander verbunden werden.

7. Verfahren nach Anspruch 6, wobei die erste und die zweite Überhangsequenz zumindest teilweise komplementär sind.

8. Verfahren nach einem der Ansprüche 4 bis 7, wobei

   a. der erste Primer für die variable Schwerekettendomäne der Reversprimer ist, der innerhalb der variablen Schwerekettendomäne der/des nativen Sequenz/Amplikons bindet, und der zweite Primer für die variable Schwerekettendomäne der Vorwärtsprimer ist, der außerhalb der variablen Schwerekettendomäne der/des nativen Sequenz/Amplikons bindet; und
   b. der erste Primer für die variable Leichtekettendomäne der Vorwärtsprimer ist, der innerhalb der variablen Leichtekettendomäne der/des nativen Sequenz/Amplikons bindet, und der zweite Primer für die variable Leichtekettendomäne der Reversprimer ist, der außerhalb der variablen Leichtekettendomäne der/des nativen Sequenz/Amplikons bindet.

9. Verfahren nach einem der Ansprüche 1 bis 8, wobei die Reagenzien Titan (Roche Kat.-Nr. 11855476001) umfassen.

10. Verfahren nach einem der Ansprüche 1 bis 9, wobei das Einkapseln die Verwendung von Mikrofluidik umfasst.

11. Verfahren nach einem der Ansprüche 1 bis 10, wobei das Einkapseln das Kombinieren einer wässrigen Suspension mit einem Öl umfasst, so dass eine Emulsion gebildet wird, die die eingekapselten Einzelzellen in Tröpfchen umfasst, wobei die wässrige Suspension die Zellen und die Reagenzien zum Amplifizieren und Verknüpfen nativer Paare von Amplikons umfasst.

12. Verfahren nach einem der Ansprüche 1 bis 11, wobei das Erzeugen der eingekapselten Amplikons die Verwendung von RT-PCR umfasst.

13. Verfahren nach einem der Ansprüche 1 bis 12, wobei das Verfahren zudem umfasst, dass es verhindert wird, dass zumindest ein Teil der freien Nukleinsäure von toten oder absterbenden Zellen in Tröpfchen eingekapselt wird.

14. Verfahren nach Anspruch 13, wobei das Verhindern das Stimulieren von Zellen für weniger als 48 Stunden vor dem Einkapseln umfasst, wobei das Verhindern das Auswählen lebender Zellen vor dem Einkapseln, oder das Sequestrieren der Nukleinsäure unter Verwendung von Oligonukleotid-beschichteten Magnetperlen umfasst.

15. Verfahren zur Herstellung einer Bibliothek nativ gepaarter Amplikons zum Screening auf Antigenbindung und/oder -funktion, wobei das Verfahren umfasst:

   a. Herstellen von eingekapselten nativ gepaarten Amplikons gemäß dem Verfahren nach einem der Ansprüche 1 bis 14; und
   b. Lysieren der Tröpfchen, um eine Bibliothek von nativ gepaarten Amplikons herzustellen, wobei jedes der scFv-Amplikons ein scFv codiert, das eine variable Schwerekettendomäne und eine variable Leichtekettendomäne umfasst, die durch einen Linker miteinander verbunden sind, wobei der Linker eine Länge von 10 bis 20 Aminosäuren aufweist.

**Revendications**

1. Procédé de production d'amplicons scFv encapsulés appariés de manière native, le procédé comprenant :

   a. l'encapsulation de lymphocytes B individuels dans des gouttelettes, les gouttelettes contenant en outre des réactifs pour amplifier et lier des appariements natifs d'amplicons de domaine variable de chaîne lourde et légère provenant de lymphocytes B individuels encapsulés ;
   b. la lyse des lymphocytes B individuels encapsulés ; et
   c. la génération des amplicons scFv encapsulés appariés de manière native, chaque amplicon scFv comprenant un appariement natif d'amplicons de domaine variable de chaîne lourde et légère liés conjointement par un lieur, le lieur ayant une longueur de 10 à 20 acides aminés.

2. Procédé selon la revendication 1, dans lequel les réactifs comprennent des amorces conçues pour des séquences d'Ig humaines.

3. Procédé selon la revendication 2, dans lequel les réactifs comprennent un groupe d'amorces comprenant des amorces ayant les séquences de SEQ ID NO : 1 à 94 ou ayant les séquences de SEQ ID NO : 1-3, 169, 76-79, 170, 171,82-91, 172,93, 94, 35, 37, 173, 174, 39, 40, 41, 175, 43, 44-46, 176, 48-54, 56-60, 177, 62, 178-184, 7-10, 185, 12, 13, 15-19, 186-189, 20-22, 190, 24, 191,26-34, 63, 192, 65, 66 et 193.

4. Procédé selon l'une quelconque des revendications 1 à 3, dans lequel la génération des amplicons encapsulés comprend initialement la formation d'amplicons de domaine variable de chaîne lourde et légère à partir de séquences natives de domaine variable de chaîne lourde et légère et les réactifs comprennent un groupe d'amorces comprenant

   a. des première et deuxième amorces de domaine variable de chaîne lourde ; et
   b. des première et deuxième amorces de domaine variable de chaîne légère,

   la première amorce de domaine variable de chaîne lourde et la première amorce de domaine variable de chaîne légère interagissent pour assembler les amplicons de domaine variable de chaîne lourde et légère.

5. Procédé selon la revendication 4, dans lequel le groupe d'amorces comprend une concentration de premières amorces plus faible que celle des deuxièmes amorces.

6. Procédé selon la revendication 4 ou la revendication 5, dans lequel la première amorce de domaine variable de chaîne lourde est fusionnée à une première séquence saillante et la première amorce de domaine variable de chaîne légère est fusionnée à une deuxième séquence saillante, dans lequel les séquences saillantes interagissent pour assembler les amplicons de domaine variable de chaîne lourde et légère.

7. Procédé selon la revendication 6, dans lequel les première et deuxième séquences saillantes sont au moins partiellement complémentaires.

8. Procédé selon l'une quelconque des revendications 4 à 7, dans lequel :

   a. la première amorce de domaine variable de chaîne lourde est l'amorce antisens qui se lie à l'intérieur du domaine variable de chaîne lourde de la séquence native/de l'amplicon, et la deuxième amorce de domaine variable de chaîne lourde est l'amorce sens qui se lie à l'extérieur du domaine variable de chaîne lourde de la séquence native/de l'amplicon ; et
b. la première amorce de domaine variable de chaîne légère est l'amorce sens qui se lie à l'intérieur du domaine variable de la chaîne légère de la séquence native/de l'amplicon, et la deuxième amorce de domaine variable de chaîne légère est l'amorce antisens qui se lie à l'extérieur du domaine variable de chaîne légère de la séquence native/de l'amplicon.

9. Procédé selon l'une quelconque des revendications 1 à 8, dans lequel les réactifs comprennent Titan (Roche référence n° 11855476001).

10. Procédé selon l'une quelconque des revendications 1 à 9, dans lequel l'encapsulation comprend l'utilisation de la microfluidique.

11. Procédé selon l'une quelconque des revendications 1 à 10, dans lequel l'encapsulation comprend la combinaison d'une suspension aqueuse avec une huile pour former une émulsion comprenant les cellules individuelles encapsulées dans des gouttelettes, dans lequel la suspension aqueuse comprend les cellules et les réactifs pour amplifier et lier les appariements natifs d'amplicons.

12. Procédé selon l'une quelconque des revendications 1 à 11, dans lequel la génération des amplicons encapsulés comprend l'utilisation de la RT-PCR.

13. Procédé selon l'une quelconque des revendications 1 à 12, le procédé comprenant en outre le blocage de l'encapsulation d'au moins une partie de l'acide nucléique libre provenant de cellules mortes ou mourantes dans des gouttelettes.

14. Procédé selon la revendication 13, dans lequel la prévention comprend la stimulation des cellules pendant moins de 48 heures avant l'encapsulation, dans lequel la prévention comprend la sélection de cellules vivantes avant l'encapsulation, ou la séquestration d'acide nucléique à l'aide de billes magnétiques enduites d'oligonucléotides.

15. Procédé de production d'une banque d'amplicons appariés de manière native pour cribler la liaison et/ou la fonction d'un antigène, le procédé comprenant

   a. la production d'amplicons encapsulés appariés de manière native selon le procédé de l'une quelconque des revendications 1 à 14 ; et
   b. la lyse des gouttelettes pour produire une bibliothèque d'amplicons appariés de manière native, chacun des amplicons scFv codant pour un scFv comprenant un domaine variable de chaîne lourde et un domaine variable de chaîne légère liés conjointement par un lieur, le lieur ayant une longueur de 10 à 20 acides aminés.

EP 3 443 087 B1

**Figure 1**

Isolated
B cells

Microfluidic
droplet
Generation

scFv

High-throughput screening

Phage-display panning

Deep sequencing

Figure 2

| Brightfield | FITC | Texas Red |

EP 3 443 087 B1

**Figure 3**

Figure 4

**Figure 5**

Figure 6

Figure 7

Figure 8

Serum Antibody Titers
for MedI donors 642 and 432

-o- 642/HA-H1SD
-△- 642/mannan

-●- 432/HA-H1SD
-▲- 432/mannan

Figure 9

Figure 10

Figure 11

**Figure 12**

Figure 13

## Figure 14A

Quality scores across all bases
(Sanger/Illumina 1.9 encoding)

## Figure 14B

Quality scores across all bases
(Sanger/Illumina 1.9 encoding)

Figure 15

# Figure 16A

# Figure 16B

## Figure 16C

# Figure 16D

# Figure 17

# Figure 18

EP 3 443 087 B1

**Table 1**

| Mix | Name | SEQ ID NO | Sequence |
|---|---|---|---|
| VH-in-R | VH_in_3_01 | 1 | gagccacctccgccgctaccgccgcctccagaGGAGACGGTGACCGTGG |
| | VH_in_3_02 | 2 | gagccacctccgccgctaccgccgcctccagaGGAGACAGTGACCAGGGTG |
| | VH_in_3_03 | 3 | gagccacctccgccgctaccgccgcctccagaGGAGACGGTGACCAGGGT |
| | VH_in_3_04 | 4 | gagccacctccgccgctaccgccgcctccagaAGAGACGGTGACCATTGTCC |
| VL-in-F | VK_in_5_01 | 5 | agcggcggaggtggctcaggcggtggcggaagtGAAATWGTGWTGACGCAGTCTCC |
| | VK_in_5_02 | 6 | agcggcggaggtggctcaggcggtggcggaagtRACATCCAGATGACCCAGTCTC |
| | VK_in_5_03 | 7 | agcggcggaggtggctcaggcggtggcggaagtGCCATCCGGATGACCCAG |
| | VK_in_5_04 | 8 | agcggcggaggtggctcaggcggtggcggaagtGAAATAGTGATGATGCAGTCTCCAG |
| | VK_in_5_05 | 9 | agcggcggaggtggctcaggcggtggcggaagtGAAACGACACTCACGCAGTC |
| | VK_in_5_06 | 10 | agcggcggaggtggctcaggcggtggcggaagtGACATCGTGATGACCCAGTCT |
| | VK_in_5_07 | 11 | agcggcggaggtggctcaggcggtggcggaagtGAGATTGTGATGACCCAGACTCC |
| | VK_in_5_08 | 12 | agcggcggaggtggctcaggcggtggcggaagtGACATCCAGTTGACCCAGTCT |
| | VK_in_5_09 | 13 | agcggcggaggtggctcaggcggtggcggaagtGTCATCTGGATGACCCAGTCTC |
| | VK_in_5_10 | 14 | agcggcggaggtggctcaggcggtggcggaagtGAAATTGTGTTGACACAGTCTCCA |
| | VK_in_5_11 | 15 | agcggcggaggtggctcaggcggtggcggaagtGATATTGTGATGACTCAGTCTCCAC |
| | VK_in_5_12 | 16 | agcggcggaggtggctcaggcggtggcggaagtGATGTTGTGATGACTCAGTCTCCA |
| | VK_in_5_13 | 17 | agcggcggaggtggctcaggcggtggcggaagtGAAATTGTAATGACACAGTCTCCAGC |
| | VK_in_5_14 | 18 | agcggcggaggtggctcaggcggtggcggaagtGCCATCCAGWTGACCCAGT |
| | VK_in_5_15 | 19 | agcggcggaggtggctcaggcggtggcggaagtGATATTGTGATGACCCAGACTCCA |
| | VL_in_5_01 | 20 | agcggcggaggtggctcaggcggtggcggaagtCAGRCTGTGGTGACYCAGG |
| | VL_in_5_02 | 21 | agcggcggaggtggctcaggcggtggcggaagtTCCTATGAGCTGACTCAGCCA |
| | VL_in_5_03 | 22 | agcggcggaggtggctcaggcggtggcggaagtCAGTCTGTGCTGACGCAG |
| | VL_in_5_04 | 23 | agcggcggaggtggctcaggcggtggcggaagtCAGGCAGGGCTGACTCAG |
| | VL_in_5_05 | 24 | agcggcggaggtggctcaggcggtggcggaagtAATTTTATGCTGACTCAGCCCC |
| | VL_in_5_06 | 25 | agcggcggaggtggctcaggcggtggcggaagtTCCTATGAGCTGAYRCAGCC |
| | VL_in_5_07 | 26 | agcggcggaggtggctcaggcggtggcggaagtCWGSCTGTGCTGACTCAGC |
| | VL_in_5_08 | 27 | agcggcggaggtggctcaggcggtggcggaagtCAGCYTGTGCTGACTCAATCR |
| | VL_in_5_09 | 28 | agcggcggaggtggctcaggcggtggcggaagtCAGTCTGCCCTGACTCAGC |
| | VL_in_5_10 | 29 | agcggcggaggtggctcaggcggtggcggaagtCAGTCTGTSKTGACGCAGC |
| | VL_in_5_11 | 30 | agcggcggaggtggctcaggcggtggcggaagtCAGACTGTGGTGACTCAGGAG |
| | VL_in_5_12 | 31 | agcggcggaggtggctcaggcggtggcggaagtCAGTCTGTGCTGACTCAGCC |
| | VL_in_5_13 | 32 | agcggcggaggtggctcaggcggtggcggaagtTCTTCTGAGCTGACTCAGGACC |
| | VL_in_5_14 | 33 | agcggcggaggtggctcaggcggtggcggaagtTCCTATGAGCTGACACAGCTAC |
| | VL_in_5_15 | 34 | agcggcggaggtggctcaggcggtggcggaagtTCCTATGTGCTGACTCAGCC |
| VH-out-F | VH_out_5_01 | 35 | AAAAGGTGTCCAATGTGAGGTGC |
| | VH_out_5_02 | 36 | AAGGAGTCTGTGCCGAGG |
| | VH_out_5_03 | 37 | AAGGTGTCCAGTGTGAGGT |

| | | | |
|---|---|---|---|
| | VH_out_5_04 | 38 | ACAGATGCCTACTCCCAGATG |
| | VH_out_5_05 | 39 | AAAGCTGTCCAGTGTCAGGT |
| | VH_out_5_06 | 40 | CAGCAGCTACAGGTGTCCA |
| | VH_out_5_07 | 41 | AAGAGGTGTCCAGTGTCAGGT |
| | VH_out_5_08 | 42 | GGTGGCAGCTCCCAGATG |
| | VH_out_5_09 | 43 | CTGACCACCCCTTCCTGG |
| | VH_out_5_10 | 44 | GTGGCAGCTCCCAGATGG |
| | VH_out_5_11 | 45 | ATGGGGTGTCCTGTCACAG |
| | VH_out_5_12 | 46 | AAGTGCCCACTCCCAGGT |
| | VH_out_5_13 | 47 | GCTATTTTAAAAGGTGTCCAGTGTG |
| | VH_out_5_14 | 48 | AGCAGCTACAGGCACCCA |
| | VH_out_5_15 | 49 | CCATGGGTGTCCTGTCACA |
| | VH_out_5_16 | 50 | ACAGGTGTCCACTCCCAGG |
| | VH_out_5_17 | 51 | ACTGACTGTCCCGTCCTGG |
| | VH_out_5_18 | 52 | ACAGGTGCCCACTCCCAG |
| | VH_out_5_19 | 53 | TGGTTCTTCCTCCTGCTGG |
| | VH_out_5_20 | 54 | CCCCTCCACAGTGAGAGTC |
| | VH_out_5_21 | 55 | ACAGGTGCCCACTCCCAAA |
| | VH_out_5_22 | 56 | AGCTCCAGGTGCTCACTCC |
| | VH_out_5_23 | 57 | CAGCCACAGGAGCCCACT |
| | VH_out_5_24 | 58 | AGGTGTCCAGTGTCAGGTG |
| | VH_out_5_25 | 59 | CTGCTGACCATCCCTTCATG |
| | VH_out_5_26 | 60 | CCTTGTTGCTATTTTAAAAGGTGTCC |
| | VH_out_5_27 | 61 | AAGGAGTCTGTTCCGAGGTG |
| | VH_out_5_28 | 62 | CTGGCTGTAGCACCAGGT |
| VL-out-R | VK_out_3_01 | 63 | CCACAGTTCGTTTRATHTCCAS |
| | VL_out_3_01 | 64 | AGAGGAGGGTGGGAACAGA |
| | VL_out_3_02 | 65 | ACTTCCACTGCTCAGGCG |
| | VL_out_3_03 | 66 | AACAGAGTGACCGAGGGG |
| | VL_out_3_04 | 67 | AGAGGAGGGCGGGAACAG |
| VL-in-R | VK_in_3_01c | 68 | cttgtgctctgcggccgcTTTGATCTCCASCTTGGTCCCTCCGCCGAAAGT |
| | VK_in_3_02c | 69 | cttgtgctctgcggccgcTTTGATTTCCACCTTGGTCCCTTGGCCGAACGT |
| | VK_in_3_03c | 70 | cttgtgctctgcggccgcTTTAATCTCCAGTCGTGTCCCTTGGCCGAAGGT |
| | VK_in_3_04c | 71 | cttgtgctctgcggccgcTTTGATATCCACTTTGGTCCCAGGGCCGAAAGT |
| | VL_in_3_01c | 72 | cttgtgctctgcggccgcTAGGACGGTCAGCTTGGTCCCTCCGCCGAAYAC |
| | VL_in_3_02c | 73 | cttgtgctctgcggccgcGAGGRCGGTCAGCTGGGTGCCTCCTCCGAACAC |
| | VL_in_3_03c | 74 | cttgtgctctgcggccgcTAGGACGGTGACCTTGGTCCCAGTTCCGAAGAC |
| | VL_in_3_05c | 75 | cttgtgctctgcggccgcGAGGACGGTCACCTTGGTGCCACTGCCGAACAC |
| VH-in-F | VH_in_5_01 | 76 | caagcagaagacggcatacgagatggcccagccggccatggccCAGGTGCAGCTACAACAGTG |
| | VH_in_5_02 | 77 | caagcagaagacggcatacgagatggcccagccggccatggccCAGGTRCAGCTRCAGSAGT |
| | VH_in_5_03 | 78 | caagcagaagacggcatacgagatggcccagccggccatggccCAGGTCACCTTGAAGGAGTCT |

| VH_in_5_04 | 79 | caagcagaagacggcatacgagatggcccagccggccatggccCAGATCACCTTGAAGGAGTCTGG |
| VH_in_5_05 | 80 | caagcagaagacggcatacgagatggcccagccggccatggccCAGGTGCAGTCTGGTGGAG |
| VH_in_5_06 | 81 | caagcagaagacggcatacgagatggcccagccggccatggccGARGTGCADCTGGTGGAGT |
| VH_in_5_07 | 82 | caagcagaagacggcatacgagatggcccagccggccatggccCAGGTYCAGCTKGTGCAGT |
| VH_in_5_08 | 83 | caagcagaagacggcatacgagatggcccagccggccatggccCAGGTACAGCTGGTGGAGTC |
| VH_in_5_09 | 84 | caagcagaagacggcatacgagatggcccagccggccatggccCGGCTGCAGCTGCAGG |
| VH_in_5_10 | 85 | caagcagaagacggcatacgagatggcccagccggccatggccCAGSTGCAGCTGCAGGA |
| VH_in_5_11 | 86 | caagcagaagacggcatacgagatggcccagccggccatggccGAGGTGCAGCTGGTGCA |
| VH_in_5_12 | 87 | caagcagaagacggcatacgagatggcccagccggccatggccSAGGTGCAGCTGTTGGAGT |
| VH_in_5_13 | 88 | caagcagaagacggcatacgagatggcccagccggccatggccSAGGTCCAGCTGGTACAGTC |
| VH_in_5_14 | 89 | caagcagaagacggcatacgagatggcccagccggccatggccCAGGTCACCTTGAGGGAGTC |
| VH_in_5_15 | 90 | caagcagaagacggcatacgagatggcccagccggccatggccCARATGCAGCTGGTGCAGT |
| VH_in_5_16 | 91 | caagcagaagacggcatacgagatggcccagccggccatggccCAGGTSCAGCTGGTGSA |
| VH_in_5_17 | 92 | caagcagaagacggcatacgagatggcccagccggccatggccGARGTGCAGCTGGTGSAG |
| VH_in_5_18 | 93 | caagcagaagacggcatacgagatggcccagccggccatggccCGGGTCACCTTGAGGGAG |
| VH_in_5_19 | 94 | caagcagaagacggcatacgagatggcccagccggccatggccCAGGTGCGGCTGCAGG |

Table 2

| Mix | Name | SEQ ID NO | Sequence |
|---|---|---|---|
| iVH_in_F | iVH_in_5_01 | 95 | CAGGTGCAGCTACAACAGTG |
| | iVH_in_5_02 | 96 | CAGGTRCAGCTRCAGSAGT |
| | iVH_in_5_03 | 97 | CAGGTCACCTTGAAGGAGTCT |
| | iVH_in_5_04 | 98 | CAGATCACCTTGAAGGAGTCTGG |
| | iVH_in_5_05 | 99 | CAGGTGCAGTCTGGTGGAG |
| | iVH_in_5_06 | 100 | GARGTGCADCTGGTGGAGT |
| | iVH_in_5_07 | 101 | CAGGTYCAGCTKGTGCAGT |
| | iVH_in_5_08 | 102 | CAGGTACAGCTGGTGGAGTC |
| | iVH_in_5_09 | 103 | CGGCTGCAGCTGCAGG |
| | iVH_in_5_10 | 104 | CAGSTGCAGCTGCAGGA |
| | iVH_in_5_11 | 105 | GAGGTGCAGCTGGTGCA |
| | iVH_in_5_12 | 106 | SAGGTGCAGCTGTTGGAGT |
| | iVH_in_5_13 | 107 | SAGGTCCAGCTGGTACAGTC |
| | iVH_in_5_14 | 108 | CAGGTCACCTTGAGGGAGTC |
| | iVH_in_5_15 | 109 | CARATGCAGCTGGTGCAGT |
| | iVH_in_5_16 | 110 | CAGGTSCAGCTGGTGSA |
| | iVH_in_5_17 | 111 | GARGTGCAGCTGGTGSAG |
| | iVH_in_5_18 | 112 | CGGGTCACCTTGAGGGAG |
| | iVH_in_5_19 | 113 | CAGGTGCGGCTGCAGG |
| iVH_in_R | iVH_in_3_01 | 114 | GGAGACGGTGACCGTGG |
| | iVH_in_3_02 | 115 | GGAGACAGTGACCAGGGTG |
| | iVH_in_3_03 | 116 | GGAGACGGTGACCAGGGT |
| | iVH_in_3_04 | 117 | AGAGACGGTGACCATTGTCC |
| iVK_in_F | iVK_in_5_01 | 118 | GAAATWGTGWTGACGCAGTCTCC |
| | iVK_in_5_02 | 119 | RACATCCAGATGACCCAGTCTC |
| | iVK_in_5_03 | 120 | GCCATCCGGATGACCCAG |
| | iVK_in_5_04 | 121 | GAAATAGTGATGATGCAGTCTCCAG |
| | iVK_in_5_05 | 122 | GAAACGACACTCACGCAGTC |
| | iVK_in_5_06 | 123 | GACATCGTGATGACCCAGTCT |
| | iVK_in_5_07 | 124 | GAGATTGTGATGACCCAGACTCC |
| | iVK_in_5_08 | 125 | GACATCCAGTTGACCCAGTCT |
| | iVK_in_5_09 | 126 | GTCATCTGGATGACCCAGTCTC |
| | iVK_in_5_10 | 127 | GAAATTGTGTTGACACAGTCTCCA |
| | iVK_in_5_11 | 128 | GATATTGTGATGACTCAGTCTCCAC |
| | iVK_in_5_12 | 129 | GATGTTGTGATGACTCAGTCTCCA |
| | iVK_in_5_13 | 130 | GAAATTGTAATGACACAGTCTCCAGC |
| | iVK_in_5_14 | 131 | GCCATCCAGWTGACCCAGT |

| | | | |
|---|---|---|---|
| | iVK_in_5_15 | 132 | GATATTGTGATGACCCAGACTCCA |
| iVK_in_R | iVK_in_3_01b | 133 | TTTGATCTCCASCTTGGTCCCTCCG |
| | iVK_in_3_02b | 134 | TTTGATTTCCACCTTGGTCCCTTGGCCGA |
| | iVK_in_3_03b | 135 | TTTAATCTCCAGTCGTGTCCCTTGGCCGA |
| | iVK_in_3_04b | 136 | TTTGATATCCACTTTGGTCCCAGGGCCGA |
| iVL_in_F | iVL_in_5_01 | 137 | CAGRCTGTGGTGACYCAGG |
| | iVL_in_5_02 | 138 | TCCTATGAGCTGACTCAGCCA |
| | iVL_in_5_03 | 139 | CAGTCTGTGCTGACGCAG |
| | iVL_in_5_04 | 140 | CAGGCAGGGCTGACTCAG |
| | iVL_in_5_05 | 141 | AATTTTATGCTGACTCAGCCCC |
| | iVL_in_5_06 | 142 | TCCTATGAGCTGAYRCAGCC |
| | iVL_in_5_07 | 143 | CWGSCTGTGCTGACTCAGC |
| | iVL_in_5_08 | 144 | CAGCYTGTGCTGACTCAATCR |
| | iVL_in_5_09 | 145 | CAGTCTGCCCTGACTCAGC |
| | iVL_in_5_10 | 146 | CAGTCTGTSKTGACGCAGC |
| | iVL_in_5_11 | 147 | CAGACTGTGGTGACTCAGGAG |
| | iVL_in_5_12 | 148 | CAGTCTGTGCTGACTCAGCC |
| | iVL_in_5_13 | 149 | TCTTCTGAGCTGACTCAGGACC |
| | iVL_in_5_14 | 150 | TCCTATGAGCTGACACAGCTAC |
| | iVL_in_5_15 | 151 | TCCTATGTGCTGACTCAGCC |
| iVL_in_R | iVL_in_3_01b | 152 | TAGGACGGTCAGCTTGGTCCCTCC |
| | iVL_in_3_02b | 153 | GAGGRCGGTCAGCTGGGTGCCTCCT |
| | iVL_in_3_03b | 154 | TAGGACGGTGACCTTGGTCCCAGTTC |
| | iVL_in_3_05b | 155 | GAGGACGGTCACCTTGGTGCCACTGCCGA |

47

Table 3

| Name | SEQ ID NO | Sequence |
|---|---|---|
| Hs-CH1-out-F | 156 | AAGGGCCCATCGGTCTTC |
| Hs-CH1-in-F | 157 | CACCCTCCTCCAAGAGCAC |
| Hs-CH1-in-R | 158 | gagccacctccgccgctaccgccgcctccagaTCTTGTCCACCTTGGTGTTG |
| Hs-CK-in-F | 159 | agcggcggaggtggctcaggcggtggcggaagtGTGGCTGCACCATCTGTCT |
| Hs-CK-out-R | 160 | TCCCCTGTTGAAGCTCTTTG |
| Hs-CK-in-R | 161 | CTGTTGAAGCTCTTTGTGACG |
| Mm-CH1-out-F | 162 | CGACACCCCCATCTGTCTAT |
| Mm-CH1-in-F | 163 | CCCCCATCTGTCTATCCACT |
| Mm-CH1-in-R | 164 | gagccacctccgccgctaccgccgcctccagaCAATTTTCTTGTCCACCTTGG |
| Mm-CK-in-F | 165 | agcggcggaggtggctcaggcggtggcggaagtCTGTATCCATCTTCCCACCA |
| Mm-CK-out-R | 166 | ACTCATTCCTGTTGAAGCTCTTG |
| Mm-CK-in-R | 167 | GGGTGAAGTTGATGTCTTGTGA |

Table 4

| Library | Donor | Colonies Picked | Binders | |
|---------|-------|-----------------|---------|---|
| | | | Influenza HA-SD | *C. albicans* mannan |
| Droplet | 432 | 1408 | 2 | 0 |
| | 642 | 1408 | 2 | 1 |
| Open | 432 | 1408 | 0 | 0 |
| | 642 | 1408 | 1 | 0 |
| | Total: | 5632 | 5 | 1 |

**Table 5**

| Primer Name | SEQ ID NO | Primer Sequence | Target V/J Gene |
|---|---|---|---|
| VH_in_3_01 | 1 | gagccacctccgccgctaccgccgcctccagaGGAGA CGGTGACCGTGG | IGHJ6*01,IGHJ6*04 |
| VH_in_3_02 | 2 | gagccacctccgccgctaccgccgcctccagaGGAGA CAGTGACCAGGGTG | IGHJ2*01 |
| VH_in_3_03 | 3 | gagccacctccgccgctaccgccgcctccagaGGAGA CGGTGACCAGGGT | IGHJ1*01,IGHJ4*01,IGHJ4*02,IGHJ4*03 ,IGHJ5*01,IGHJ5*02 |
| VH_in_3_04h | 169 | gagccacctccgccgctaccgccgcctccagaAGAGA CGRTGACCATTGTCC | IGHJ3*01,IGHJ3*02 |
| VH_in_5_01f | 76 | caagcagaagacggcatacgagatggcccagccggcca tggccCAGGTGCAGCTACAACAGTG | IGHV4-34*02 |
| VH_in_5_02f | 77 | caagcagaagacggcatacgagatggcccagccggcca tggccCAGGTRCAGCTRCAGSAGT | IGHV4-28*01,IGHV4-28*02,IGHV4-28*03,IGHV4-28*04,IGHV4-28*05,IGHV4-28*06,IGHV4-28*07,IGHV4-30-4*01,IGHV4-30-4*02,IGHV4-30-4*03,IGHV4-30-4*07,IGHV4-31*01,IGHV4-31*02,IGHV4-31*03,IGHV4-31*05,IGHV4-31*06,IGHV4-31*07,IGHV4-31*08,IGHV4-31*09,IGHV4-31*10,IGHV4-34*01,IGHV4-34*03,IGHV4-34*04,IGHV4-34*05,IGHV4-34*06,IGHV4-34*07,IGHV4-34*08,IGHV4-34*09,IGHV4-34*10,IGHV4-34*11,IGHV4-34*12,IGHV4-38-2*01,IGHV4-38-2*02,IGHV4-4*01,IGHV4-4*02,IGHV4-4*03,IGHV4-4*04,IGHV4-4*05,IGHV4-4*07,IGHV4-4*08,IGHV4-59*01,IGHV4-59*02,IGHV4-59*03,IGHV4-59*04,IGHV4-59*05,IGHV4-59*06,IGHV4-59*07,IGHV4-59*08,IGHV4-59*10,IGHV4-61*01,IGHV4-61*02,IGHV4-61*03,IGHV4-61*04,IGHV4-61*08,IGHV6-1*01,IGHV6-1*02 |
| VH_in_5_03f | 78 | caagcagaagacggcatacgagatggcccagccggcca tggccCAGGTCACCTTGAAGGAGTCT | IGHV2-26*01,IGHV2-5*08,IGHV2-5*09,IGHV2-70*03,IGHV2-70*04,IGHV2-70*06,IGHV2-70*10,IGHV2-70D*04,IGHV2-70D*14 |

| Primer Name | SEQ ID NO | Primer Sequence | Target V/J Gene |
|---|---|---|---|
| VH_in_5_04f | 79 | caagcagaagacggcatacgagatggcccagccggcca tggccCAGATCACCTTGAAGGAGTCTGG | IGHV2-5*01,IGHV2-5*02,IGHV2-5*04,IGHV2-5*05,IGHV2-5*06,IGHV2-70*09,IGHV2-70*12 |
| VH_in_5_05f | 170 | caagcagaagacggcatacgagatggcccagccggcca tggccCAGGTGCAGTCTGGTGGAGT | IGHV3-11*02 |
| VH_in_5_06f | 171 | caagcagaagacggcatacgagatggcccagccggcca tggccGARGTGCADCTGGTGGAGWC | IGHV3-13*01,IGHV3-13*02,IGHV3-13*03,IGHV3-13*04,IGHV3-13*05,IGHV3-15*01,IGHV3-15*02,IGHV3-15*03,IGHV3-15*04,IGHV3-15*05,IGHV3-15*06,IGHV3-15*07,IGHV3-15*08,IGHV3-20*01,IGHV3-20*02,IGHV3-21*01,IGHV3-21*02,IGHV3-21*03,IGHV3-21*04,IGHV3-23*04,IGHV3-43*01,IGHV3-43*02,IGHV3-43D*01,IGHV3-48*01,IGHV3-48*02,IGHV3-48*03,IGHV3-48*04,IGHV3-49*01,IGHV3-49*02,IGHV3-49*03,IGHV3-49*04,IGHV3-49*05,IGHV3-53*01,IGHV3-53*02,IGHV3-53*03,IGHV3-53*04,IGHV3-64*01,IGHV3-64*02,IGHV3-64*03,IGHV3-64*05,IGHV3-64D*06,IGHV3-66*01,IGHV3-66*02,IGHV3-66*03,IGHV3-66*04,IGHV3-7*01,IGHV3-7*02,IGHV3-7*03,IGHV3-72*01,IGHV3-73*01,IGHV3-73*02,IGHV3-74*01,IGHV3-74*02,IGHV3-74*03,IGHV3-9*01,IGHV3-9*02,IGHV3-9*03 |
| VH_in_5_07f | 82 | caagcagaagacggcatacgagatggcccagccggcca tggccCAGGTYCAGCTKGTGCAGT | IGHV1-18*01,IGHV1-18*02,IGHV1-18*03,IGHV1-18*04,IGHV1-3*01,IGHV1-3*02,IGHV1-69*04,IGHV1-69*05,IGHV1-69*10,IGHV1-69*11,IGHV1-69*12,IGHV1-69*13,IGHV1-69*14 |
| VH_in_5_08f | 83 | caagcagaagacggcatacgagatggcccagccggcca tggccCAGGTACAGCTGGTGGAGTC | IGHV3-33*02 |
| VH_in_5_09f | 84 | caagcagaagacggcatacgagatggcccagccggcca tggccCGGCTGCAGCTGCAGG | IGHV4-39*06 |

| Primer Name | SEQ ID NO | Primer Sequence | Target V/J Gene |
|---|---|---|---|
| VH_in_5_10f | 85 | caagcagaagacggcatacgagatggcccagccggcca tggccCAGSTGCAGCTGCAGGA | IGHV4-28*01,IGHV4-28*02,IGHV4-28*03,IGHV4-28*04,IGHV4-28*05,IGHV4-30-2*01,IGHV4-30-2*02,IGHV4-30-2*03,IGHV4-30-2*05,IGHV4-30-2*06,IGHV4-30-4*01,IGHV4-30-4*02,IGHV4-30-4*03,IGHV4-30-4*04,IGHV4-30-4*07,IGHV4-31*01,IGHV4-31*02,IGHV4-31*03,IGHV4-31*05,IGHV4-31*06,IGHV4-31*07,IGHV4-31*08,IGHV4-31*09,IGHV4-31*10,IGHV4-34*09,IGHV4-34*10,IGHV4-38-2*01,IGHV4-38-2*02,IGHV4-39*01,IGHV4-39*02,IGHV4-39*03,IGHV4-39*05,IGHV4-39*07,IGHV4-4*01,IGHV4-4*02,IGHV4-4*03,IGHV4-4*04,IGHV4-4*05,IGHV4-4*07,IGHV4-4*08,IGHV4-59*01,IGHV4-59*02,IGHV4-59*03,IGHV4-59*04,IGHV4-59*05,IGHV4-59*06,IGHV4-59*07,IGHV4-59*08,IGHV4-61*01,IGHV4-61*02,IGHV4-61*03,IGHV4-61*04,IGHV4-61*05,IGHV4-61*08 |
| VH_in_5_11f | 86 | caagcagaagacggcatacgagatggcccagccggcca tggccGAGGTGCAGCTGGTGCA | IGHV5-51*01,IGHV5-51*02,IGHV5-51*03,IGHV5-51*04 |
| VH_in_5_12f | 87 | caagcagaagacggcatacgagatggcccagccggcca tggccSAGGTGCAGCTGTTGGAGT | IGHV3-11*03,IGHV3-23*01,IGHV3-23*02,IGHV3-23*03,IGHV3-23*05,IGHV3-23D*01 |
| VH_in_5_13f | 88 | caagcagaagacggcatacgagatggcccagccggcca tggccSAGGTCCAGCTGGTACAGTC | IGHV1-24*01,IGHV1-69-2*01 |
| VH_in_5_14f | 89 | caagcagaagacggcatacgagatggcccagccggcca tggccCAGGTCACCTTGAGGGAGTC | IGHV2-70*01,IGHV2-70*02,IGHV2-70*07,IGHV2-70*08,IGHV2-70*13 |
| VH_in_5_15f | 90 | caagcagaagacggcatacgagatggcccagccggcca tggccCARATGCAGCTGGTGCAGT | IGHV1-45*01,IGHV1-45*02,IGHV1-58*01,IGHV1-58*02 |

| Primer Name | SEQ ID NO | Primer Sequence | Target V/J Gene |
|---|---|---|---|
| VH_in_5_16f | 91 | caagcagaagacggcatacgagatggcccagccggcca tggccCAGGTSCAGCTGGTGSA | IGHV1-2*01,IGHV1-2*02,IGHV1-2*03,IGHV1-2*04,IGHV1-2*05,IGHV1-46*01,IGHV1-46*02,IGHV1-46*03,IGHV1-69*01,IGHV1-69*02,IGHV1-69*03,IGHV1-69*04,IGHV1-69*05,IGHV1-69*06,IGHV1-69*08,IGHV1-69*09,IGHV1-69*10,IGHV1-69*11,IGHV1-69*12,IGHV1-69*13,IGHV1-69*14,IGHV1-69D*01,IGHV1-8*01,IGHV1-8*02,IGHV3-11*01,IGHV3-11*04,IGHV3-11*05,IGHV3-11*06,IGHV3-30*01,IGHV3-30*02,IGHV3-30*03,IGHV3-30*04,IGHV3-30*05,IGHV3-30*06,IGHV3-30*07,IGHV3-30*08,IGHV3-30*09,IGHV3-30*10,IGHV3-30*11,IGHV3-30*12,IGHV3-30*13,IGHV3-30*14,IGHV3-30*15,IGHV3-30*16,IGHV3-30*17,IGHV3-30*18,IGHV3-30*19,IGHV3-30-3*01,IGHV3-30-3*02,IGHV3-30-3*03,IGHV3-33*01,IGHV3-33*03,IGHV3-33*04,IGHV3-33*05,IGHV3-33*06,IGHV3-64*04,IGHV3-NL1*01,IGHV7-4-1*01,IGHV7-4-1*02,IGHV7-4-1*04,IGHV7-4-1*05 |
| VH_in_5_17f | 172 | caagcagaagacggcatacgagatggcccagccggcca tggccGAAGTGCAGCTGGTGCAGT | IGHV5-10-1*01,IGHV5-10-1*02,IGHV5-10-1*03,IGHV5-10-1*04 |
| VH_in_5_18f | 93 | caagcagaagacggcatacgagatggcccagccggcca tggccCGGGTCACCTTGAGGGAG | IGHV2-70*11 |
| VH_in_5_19f | 94 | caagcagaagacggcatacgagatggcccagccggcca tggccCAGGTGCGGCTGCAGG | IGHV4-31*04 |
| VH_out_5_01 | 35 | AAAAGGTGTCCAATGTGAGGTGC | IGHV3-49*01_ldr,IGHV3-49*02_ldr,IGHV3-49*03_ldr |
| VH_out_5_03 | 37 | AAGGTGTCCAGTGTGAGGT | IGHV3-13*01_ldr,IGHV3-13*02_ldr,IGHV3-15*01_ldr,IGHV3-15*02_ldr,IGHV3-15*03_ldr,IGHV3-15*04_ldr,IGHV3-15*05_ldr,IGHV3-15*06_ldr,IGHV3-15*07_ldr,IGHV3-15*08_ldr,IGHV3-20*01_ldr,IGHV3-21*01_ldr,IGHV3-21*02_ldr,IGHV3-23*01_ldr,IGHV3-23*02_ldr,IGHV3-23*04_ldr,IGHV3-48*01_ldr,IGHV3-48*02_ldr,IGHV3-48*03_ldr,IGHV3- |

| Primer Name | SEQ ID NO | Primer Sequence | Target V/J Gene |
|---|---|---|---|
| | | | 53*01_ldr,IGHV3-53*03_ldr,IGHV3-64*01_ldr,IGHV3-64*02_ldr,IGHV3-66*01_ldr,IGHV3-66*03_ldr,IGHV3-66*04_ldr,IGHV3-7*01_ldr,IGHV3-72*01_ldr,IGHV3-73*01_ldr,IGHV3-73*02_ldr,IGHV3-74*01_ldr,IGHV3-74*03_ldr |
| VH_out_5_04 | 173 | ACAGGTGYCCACTCCCARR | IGHV1-18*01_ldr,IGHV1-18*02_ldr,IGHV1-3*01_ldr,IGHV1-3*02_ldr,IGHV1-58*01_ldr,IGHV1-58*02_ldr,IGHV1-69*03_ldr,IGHV7-4-1*02_ldr |
| VH_out_5_05 | 174 | ACAGATGCCTACTCCCAGATGC | IGHV1-45*02_ldr |
| VH_out_5_06 | 39 | AAAGCTGTCCAGTGTCAGGT | IGHV3-11*02_ldr |
| VH_out_5_07 | 40 | CAGCAGCTACAGGTGTCCA | IGHV1-69*01_ldr,IGHV1-69*03_ldr,IGHV1-69*04_ldr,IGHV1-69*06_ldr |
| VH_out_5_08 | 41 | AAGAGGTGTCCAGTGTCAGGT | IGHV3-30*01_ldr,IGHV3-30*03_ldr,IGHV3-30*18_ldr,IGHV3-33*01_ldr,IGHV3-33*02_ldr |
| VH_out_5_10 | 175 | GGTGGCAGCTCCCAGATGG | IGHV4-28*01_ldr,IGHV4-28*02_ldr,IGHV4-28*03_ldr,IGHV4-30-2*03_ldr,IGHV4-30-4*01_ldr,IGHV4-30-4*02_ldr,IGHV4-30-4*03_ldr,IGHV4-30-4*04_ldr,IGHV4-31*02_ldr,IGHV4-31*03_ldr,IGHV4-31*06_ldr,IGHV4-31*07_ldr,IGHV4-31*08_ldr,IGHV4-31*09_ldr,IGHV4-31*10_ldr,IGHV4-34*01_ldr,IGHV4-34*02_ldr,IGHV4-34*03_ldr,IGHV4-34*04_ldr,IGHV4-34*05_ldr,IGHV4-34*06_ldr,IGHV4-34*07_ldr,IGHV4-34*09_ldr,IGHV4-34*10_ldr,IGHV4-34*11_ldr,IGHV4-4*01_ldr,IGHV4-4*02_ldr,IGHV4-4*03_ldr,IGHV4-4*04_ldr,IGHV4-4*05_ldr,IGHV4-4*07_ldr,IGHV4-59*01_ldr,IGHV4-59*02_ldr,IGHV4-59*10_ldr,IGHV4-61*01_ldr,IGHV4-61*03_ldr,IGHV4-61*04_ldr,IGHV4-61*08_ldr |
| VH_out_5_11 | 43 | CTGACCACCCCTTCCTGG | IGHV2-26*01_ldr |

54

| Primer Name | SEQ ID NO | Primer Sequence | Target V/J Gene |
|---|---|---|---|
| VH_out_5_12 | 44 | GTGGCAGCTCCCAGATGG | IGHV4-28*01_ldr,IGHV4-28*02_ldr,IGHV4-28*03_ldr,IGHV4-30-2*03_ldr,IGHV4-30-4*01_ldr,IGHV4-30-4*02_ldr,IGHV4-30-4*03_ldr,IGHV4-30-4*04_ldr,IGHV4-31*02_ldr,IGHV4-31*03_ldr,IGHV4-31*06_ldr,IGHV4-31*07_ldr,IGHV4-31*08_ldr,IGHV4-31*09_ldr,IGHV4-31*10_ldr,IGHV4-34*01_ldr,IGHV4-34*02_ldr,IGHV4-34*03_ldr,IGHV4-34*04_ldr,IGHV4-34*05_ldr,IGHV4-34*06_ldr,IGHV4-34*07_ldr,IGHV4-34*09_ldr,IGHV4-34*10_ldr,IGHV4-34*11_ldr,IGHV4-4*01_ldr,IGHV4-4*02_ldr,IGHV4-4*03_ldr,IGHV4-4*04_ldr,IGHV4-4*05_ldr,IGHV4-4*07_ldr,IGHV4-59*01_ldr,IGHV4-59*02_ldr,IGHV4-59*10_ldr,IGHV4-61*01_ldr,IGHV4-61*03_ldr,IGHV4-61*04_ldr,IGHV4-61*08_ldr |
| VH_out_5_13 | 45 | ATGGGGTGTCCTGTCACAG | IGHV6-1*01_ldr |
| VH_out_5_14 | 46 | AAGTGCCCACTCCCAGGT | IGHV1-8*01_ldr |
| VH_out_5_15 h | 176 | GCTATTTTAAAAGGTGTCCAGWGTG | IGHV3-15*01_ldr,IGHV3-15*02_ldr,IGHV3-15*03_ldr,IGHV3-15*04_ldr,IGHV3-15*05_ldr,IGHV3-15*06_ldr,IGHV3-15*07_ldr,IGHV3-15*08_ldr,IGHV3-20*01_ldr,IGHV3-23*02_ldr,IGHV3-23*04_ldr,IGHV3-43*01_ldr,IGHV3-53*01_ldr,IGHV3-53*03_ldr,IGHV3-66*01_ldr,IGHV3-66*03_ldr,IGHV3-66*04_ldr,IGHV3-73*01_ldr,IGHV3-73*02_ldr,IGHV3-74*01_ldr,IGHV3-74*03_ldr,IGHV3-9*01_ldr |
| VH_out_5_16 | 48 | AGCAGCTACAGGCACCCA | IGHV1-24*01_ldr |
| VH_out_5_17 | 49 | CCATGGGTGTCCTGTCACA | IGHV6-1*02_ldr |
| VH_out_5_18 | 51 | ACTGACTGTCCCGTCCTGG | IGHV2-5*08_ldr,IGHV2-5*09_ldr,IGHV2-70*01_ldr,IGHV2-70*02_ldr,IGHV2-70*03_ldr,IGHV2-70*06_ldr,IGHV2-70*07_ldr,IGHV2-70*08_ldr,IGHV2-70*11_ldr,IGHV2-70*12_ldr,IGHV2-70*13_ldr |
| VH_out_5_19 | 52 | ACAGGTGCCCACTCCCAG | IGHV1-18*01_ldr,IGHV1-18*02_ldr,IGHV1-3*01_ldr,IGHV7-4- |

| Primer Name | SEQ ID NO | Primer Sequence | Target V/J Gene |
|---|---|---|---|
| | | | 1*02_ldr |
| VH_out_5_20 | 53 | TGGTTCTTCCTCCTGCTGG | IGHV4-28*01_ldr,IGHV4-28*02_ldr,IGHV4-28*03_ldr,IGHV4-30-2*03_ldr,IGHV4-30-4*01_ldr,IGHV4-30-4*02_ldr,IGHV4-30-4*03_ldr,IGHV4-30-4*04_ldr,IGHV4-31*02_ldr,IGHV4-31*03_ldr,IGHV4-31*06_ldr,IGHV4-31*07_ldr,IGHV4-31*08_ldr,IGHV4-31*09_ldr,IGHV4-31*10_ldr,IGHV4-34*09_ldr,IGHV4-39*01_ldr,IGHV4-39*02_ldr,IGHV4-39*03_ldr,IGHV4-39*06_ldr,IGHV4-4*07_ldr |
| VH_out_5_21 | 54 | CCCCTCCACAGTGAGAGTC | IGHV5-a*02_ldr |
| VH_out_5_22 | 56 | AGCTCCAGGTGCTCACTCC | IGHV1-46*01_ldr,IGHV1-46*03_ldr |
| VH_out_5_23 | 57 | CAGCCACAGGAGCCCACT | IGHV1-2*01_ldr,IGHV1-2*02_ldr,IGHV1-2*03_ldr |
| VH_out_5_24 | 58 | AGGTGTCCAGTGTCAGGTG | IGHV3-11*01_ldr,IGHV3-30*01_ldr,IGHV3-30*03_ldr,IGHV3-30*18_ldr,IGHV3-33*01_ldr |
| VH_out_5_25 | 59 | CTGCTGACCATCCCTTCATG | IGHV2-5*01_ldr,IGHV2-5*04_ldr,IGHV2-5*05_ldr,IGHV2-5*06_ldr,IGHV2-5*07_ldr,IGHV2-70*09_ldr,IGHV2-70*10_ldr |
| VH_out_5_26 | 60 | CCTTGTTGCTATTTTAAAAGGTGTCC | IGHV3-20*01_ldr,IGHV3-43*01_ldr,IGHV3-49*01_ldr,IGHV3-49*03_ldr,IGHV3-53*01_ldr,IGHV3-53*03_ldr,IGHV3-66*01_ldr,IGHV3-66*03_ldr,IGHV3-66*04_ldr,IGHV3-73*01_ldr,IGHV3-73*02_ldr,IGHV3-74*01_ldr,IGHV3-74*03_ldr |
| VH_out_5_27 | 177 | AAGGAGTCTGTKCCGAGGTG | IGHV5-51*01_ldr,IGHV5-51*02_ldr |
| VH_out_5_28 | 62 | CTGGCTGTAGCACCAGGT | IGHV1-46*02_ldr |
| VK_in_3_01g | 178 | gattacgccaagcttggagccgcggccgcTTTGATCTCCACCTTGGTCCCTCCGCCGAAMGT | IGKJ4*01,IGKJ4*02 |
| VK_in_3_02g | 179 | gattacgccaagcttggagccgcggccgcTTTGATTTCCACCTTGGTCCCTTGGCCGAACGT | IGKJ1*01 |
| VK_in_3_03g | 180 | gattacgccaagcttggagccgcggccgcTTTAATCTCCAGTCGTGTCCCTTGGCCGAAGGT | IGKJ5*01 |
| VK_in_3_04g | 181 | gattacgccaagcttggagccgcggccgcTTTGATAT | IGKJ3*01 |

| Primer Name | SEQ ID NO | Primer Sequence | Target V/J Gene |
|---|---|---|---|
| | | CCACTTTGGTCCCAGGGCCGAAAGT | |
| VK_in_3_05g | 182 | gattacgccaagcttggagccgcggccgcTTTGATCTCCAGCTTGGTCCCCTGGCCAAAAST | IGKJ2*01,IGKJ2*02,IGKJ2*03,IGKJ2*04 |
| VK_in_5_01e | 183 | agcggcggaggtggctcaggcggtggcggaagtGAAATWGTGWTGACRCAGTCTCCA | IGKV3-11*01,IGKV3-11*02,IGKV3-15*01,IGKV3-20*01,IGKV3-20*02,IGKV3-NL1*01,IGKV3-NL2*01,IGKV3-NL3*01,IGKV3-NL4*01,IGKV3-NL5*01,IGKV3D-11*01,IGKV3D-15*01,IGKV3D-20*01 |
| VK_in_5_02h | 184 | agcggcggaggtggctcaggcggtggcggaagtRACATCCAGATGACCCAGTYTC | IGKV1-12*01,IGKV1-12*02,IGKV1-16*01,IGKV1-16*02,IGKV1-17*01,IGKV1-17*02,IGKV1-27*01,IGKV1-33*01,IGKV1-39*01,IGKV1-39*02,IGKV1-5*01,IGKV1-5*02,IGKV1-5*03,IGKV1-NL1*01,IGKV1D-12*01,IGKV1D-12*02,IGKV1D-16*01,IGKV1D-16*02,IGKV1D-17*01,IGKV1D-17*02,IGKV1D-33*01,IGKV1D-39*01 |
| VK_in_5_03 | 7 | agcggcggaggtggctcaggcggtggcggaagtGCCATCCGGATGACCCAG | IGKV1-8*01,IGKV1D-43*01 |
| VK_in_5_04 | 8 | agcggcggaggtggctcaggcggtggcggaagtGAAATAGTGATGATGCAGTCTCCAG | IGKV3D-15*02 |
| VK_in_5_05 | 9 | agcggcggaggtggctcaggcggtggcggaagtGAAACGACACTCACGCAGTC | IGKV5-2*01 |
| VK_in_5_06 | 10 | agcggcggaggtggctcaggcggtggcggaagtGACATCGTGATGACCCAGTCT | IGKV4-1*01 |
| VK_in_5_07e | 185 | agcggcggaggtggctcaggcggtggcggaagtGAGATTGTGATGACCCAGACTCCA | IGKV2D-26*01,IGKV2D-26*02 |
| VK_in_5_08 | 12 | agcggcggaggtggctcaggcggtggcggaagtGACATCCAGTTGACCCAGTCT | IGKV1-9*01 |
| VK_in_5_09 | 13 | agcggcggaggtggctcaggcggtggcggaagtGTCATCTGGATGACCCAGTCTC | IGKV1D-8*01 |
| VK_in_5_11 | 15 | agcggcggaggtggctcaggcggtggcggaagtGATATTGTGATGACTCAGTCTCCAC | IGKV2-28*01,IGKV2D-28*01 |
| VK_in_5_12 | 16 | agcggcggaggtggctcaggcggtggcggaagtGATGTTGTGATGACTCAGTCTCCA | IGKV2-30*01,IGKV2-30*02,IGKV2D-30*01 |
| VK_in_5_13 | 17 | agcggcggaggtggctcaggcggtggcggaagtGAAATTGTAATGACACAGTCTCCAGC | IGKV3D-7*01 |

57

| Primer Name | SEQ ID NO | Primer Sequence | Target V/J Gene |
|---|---|---|---|
| VK_in_5_14 | 18 | agcggcggaggtggctcaggcggtggcggaagtGCCATCCAGWTGACCCAGT | IGKV1-13*01,IGKV1-13*02,IGKV1-6*01,IGKV1D-13*01 |
| VK_in_5_15 | 19 | agcggcggaggtggctcaggcggtggcggaagtGATATTGTGATGACCCAGACTCCA | IGKV2-24*01,IGKV2-29*01,IGKV2-29*02,IGKV2-29*03,IGKV2-40*01,IGKV2D-29*01,IGKV2D-29*02,IGKV2D-40*01 |
| VL_in_3_01g | 186 | gattacgccaagcttggagccgcggccgcTAGGACGGTCAGCTTGGTCCCTCCGCCGAAYAC | IGLJ2*01,IGLJ3*01,IGLJ3*02 |
| VL_in_3_02g | 187 | gattacgccaagcttggagccgcggccgcGAGGRCGGTCAGCTGGGTGCCTCCTCCGAACAC | IGLJ7*01,IGLJ7*02 |
| VL_in_3_03g | 188 | gattacgccaagcttggagccgcggccgcTAGGACGGTGACCTTGGTCCCAGTTCCGAAGAC | IGLJ1*01 |
| VL_in_3_05g | 189 | gattacgccaagcttggagccgcggccgcGAGGACGGTCACCTTGGTGCCACTGCCGAACAC | IGLJ6*01 |
| VL_in_5_01 | 20 | agcggcggaggtggctcaggcggtggcggaagtCAGRCTGTGGTGACYCAGG | IGLV7-43*01,IGLV7-46*01,IGLV7-46*02,IGLV7-46*03,IGLV8-61*01,IGLV8-61*02 |
| VL_in_5_02 | 21 | agcggcggaggtggctcaggcggtggcggaagtTCCTATGAGCTGACTCAGCCA | IGLV3-1*01,IGLV3-12*01,IGLV3-12*02,IGLV3-9*01,IGLV3-9*02,IGLV3-9*03 |
| VL_in_5_03 | 22 | agcggcggaggtggctcaggcggtggcggaagtCAGTCTGTGCTGACGCAG | IGLV1-40*01 |
| VL_in_5_04e | 190 | agcggcggaggtggctcaggcggtggcggaagtCAGGCAGGGCTGACTCAGCCA | IGLV10-54*01,IGLV10-54*02,IGLV10-54*03 |
| VL_in_5_05 | 24 | agcggcggaggtggctcaggcggtggcggaagtAATTTTATGCTGACTCAGCCCC | IGLV6-57*01 |
| VL_in_5_06e | 191 | agcggcggaggtggctcaggcggtggcggaagtTCCTATGAGCTGAYRCAGCCAYC | IGLV3-10*01,IGLV3-10*02,IGLV3-16*01,IGLV3-25*01,IGLV3-25*02,IGLV3-25*03,IGLV3-27*01 |
| VL_in_5_07 | 26 | agcggcggaggtggctcaggcggtggcggaagtCWGSCTGTGCTGACTCAGC | IGLV4-3*01,IGLV5-37*01,IGLV5-39*01,IGLV5-39*02,IGLV5-45*01,IGLV5-45*02,IGLV5-45*03,IGLV5-52*01,IGLV9-49*01,IGLV9-49*02,IGLV9-49*03 |
| VL_in_5_08 | 27 | agcggcggaggtggctcaggcggtggcggaagtCAGCYTGTGCTGACTCAATCR | IGLV4-60*01,IGLV4-60*02,IGLV4-60*03,IGLV4-69*01,IGLV4-69*02 |
| VL_in_5_09 | 28 | agcggcggaggtggctcaggcggtggcggaagtCAGTCTGCCCTGACTCAGC | IGLV2-11*01,IGLV2-11*02,IGLV2-14*01,IGLV2-14*02,IGLV2-18*01,IGLV2-18*02,IGLV2-18*03,IGLV2-18*04,IGLV2-23*01,IGLV2-23*02,IGLV2-23*03,IGLV2- |

| Primer Name | SEQ ID NO | Primer Sequence | Target V/J Gene |
|---|---|---|---|
|  |  |  | 8*01,IGLV2-8*02 |
| VL_in_5_10 | 29 | agcggcggaggtggctcaggcggtggcggaagtCAGT CTGTSKTGACGCAGC | IGLV1-40*02,IGLV1-40*03,IGLV1-51*01,IGLV1-51*02 |
| VL_in_5_11 | 30 | agcggcggaggtggctcaggcggtggcggaagtCAGA CTGTGGTGACTCAGGAG | IGLV7-43*01 |
| VL_in_5_12 | 31 | agcggcggaggtggctcaggcggtggcggaagtCAGT CTGTGCTGACTCAGCC | IGLV1-36*01,IGLV1-44*01,IGLV1-47*01,IGLV1-47*02 |
| VL_in_5_13 | 32 | agcggcggaggtggctcaggcggtggcggaagtTCTT CTGAGCTGACTCAGGACC | IGLV3-19*01 |
| VL_in_5_14 | 33 | agcggcggaggtggctcaggcggtggcggaagtTCCT ATGAGCTGACACAGCTAC | IGLV3-22*01,IGLV3-22*02 |
| VL_in_5_15 | 34 | agcggcggaggtggctcaggcggtggcggaagtTCCT ATGTGCTGACTCAGCC | IGLV3-21*01,IGLV3-21*02,IGLV3-21*03 |
| VK_out_3_01 | 63 | CCACAGTTCGTTTRATHTCCAS | IGKC*01,IGKC*02,IGKC*03,IGKC*04,IGKC*05 |
| VL_out_3_01 | 192 | AGAGGAGGGTGGGAACAGAG | IGLC3*01,IGLC3*02,IGLC3*03,IGLC7*01,IGLC7*02 |
| VL_out_3_02 | 65 | ACTTCCACTGCTCAGGCG | IGLC2*01,IGLC2*02,IGLC2*03,IGLC3*01,IGLC3*02,IGLC3*03,IGLC3*04,IGLC6*01,IGLC6*02,IGLC6*03,IGLC6*04,IGLC6*05 |
| VL_out_3_03 | 66 | AACAGAGTGACCGAGGGG | IGLC2*01,IGLC2*02,IGLC3*01,IGLC3*02,IGLC3*03,IGLC3*04,IGLC6*02,IGLC6*05,IGLC7*01 |
| VL_out_3_04 | 193 | AGAGGAGGGCGGGAACAGAG | IGLC1*01,IGLC1*02,IGLC2*01,IGLC2*02,IGLC3*04,IGLC6*01,IGLC6*02,IGLC6*03,IGLC6*04,IGLC6*05 |

Table 6

| Primer Name | SEQ ID NO | Primer Sequence |
|---|---|---|
| Illu_R_N505 | 194 | aatgatacggcgaccaccgagatctacacGTAAGGAGgattacgccaagctttggagcc |
| Illu_R_N506 | 195 | aatgatacggcgaccaccgagatctacacACTGCATAgattacgccaagctttggagcc |
| Illu_R_N507 | 196 | aatgatacggcgaccaccgagatctacacAAGGAGTAgattacgccaagctttggagcc |
| Illu_R_N508 | 197 | aatgatacggcgaccaccgagatctacacCTAAGCCTgattacgccaagctttggagcc |
| R1 | 198 | GATTACGCCAAGCTTTGGAGCC |
| R2 | 199 | CGCTACCGCCGCCTCCAGA |
| Illu_F_scaleup | 200 | CAAGCAGAAGACGGCATACGAGAT |

Table 7

| Library | Donor 1 droplet | Donor 1 combinatorial | Donor 2 droplet | Donor 2 combinatorial |
|---|---|---|---|---|
| Mapped Reads ($V_H$) | 3,311,883 | 1,867,114 | 2,841,606 | 3,534,010 |
| Mapped Reads ($V_L$) | 2,397,908 | 898,120 | 1,981,098 | 2,553,130 |
| Mapped Reads ($V_H$-$V_L$) | 2,649,093 | 981,684 | 2,171,741 | 2,645,525 |
| Unique CDR-H3:CDR-L3 | 274,979 | 931,779 | 206,225 | 2,171,198 |
| CDR-H3 clusters | 152,032 | 445,249 | 101,488 | 376,885 |
| CDR-L3 clusters | 45,444 | 160,243 | 38,326 | 176,520 |
| CDR-H3:CDR-L3 clusters | 121,845 | 766,146 | 90,173 | 1,783,269 |
| CDR-H3:CDR-L3 adjusted Chao | 170,886 | N/A | 122,309 | N/A |
| $V_L$:$V_H$ ratio (median) | 2 | 9 | 2 | 5 |

Table 8

| p-value | SRR1585267 | SRR1585265 | SRR1585248 | SRR1585249 |
|---------|------------|------------|------------|------------|
| Donor 1 | 1.46E-157 | 1.08E-42 | 6.13E-45 | 1.30E-06 |
| Donor 2 | 3.41E-65 | 3.24E-20 | 4.14E-19 | 0.00065 |

Table 9

| Phage Library | B cell Source | Library Size | Clones Displaying scFv (%) |
|---|---|---|---|
| Donor 1 Emulsion | Total B Cells | $3.0 \times 10^8$ | 98.9 |
| Donor 1 Combinatorial | Total B Cells | $3.0 \times 10^8$ | 94.7 |
| Donor 2 Emulsion | Memory B Cells | $3.5 \times 10^8$ | 90.5 |
| Donor 2 Combinatorial | Memory B Cells | $1.0 \times 10^8$ | 95.7 |

## EP 3 443 087 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DEKOSKY, B.J. et al.** *Nat. Biotechnol.,* 2013, vol. 31, 166-169
- **STERN J.** *Sci Trans. Med.,* 2014, vol. 6, 248
- **TAN Y-C.** *Arthritis. Rheumatol.,* 2014, vol. 66, 2706-2715
- **TAN Y-C.** *Clin. Immunol,* 2014, vol. 151, 55-65
- **LU, D.R.** *Clin. Immunol.,* 2014, vol. 152, 77-89
- **ROBINS, W.H.** *Curr. Opin. Immunol.,* 2013, vol. 25, 646-652
- **DEKOSKY, B. J. et al.** *Nat. Med.,* 2015, vol. 21, 86-91
- **GALSON, J. D. et al.** *Crit. Rev. Immunol.,* 2015, vol. 35, 463-478
- **MEIJER, P. J et al.** *J.Mol.Biol.,* 2006, vol. 358, 764-772
- **TILLER, T.** *J Immunol. Methods,* 2008, vol. 329, 112-124
- **LEVIN et al.** *J Allergy Clin Immunol.,* May 2016, vol. 137 (5), 1535-44
- **TURCHANINOVA et al.** *Eur J Immunol.,* September 2013, vol. 43 (9), 2507-15
- **DEKOSKY et al.** *Nature Biotechnology,* 2013, vol. 31, 166-169
- **DEKOSKY, B.J. et al.** *Nat. Med.,* 2015, vol. 21, 86-91
- **WHITE, A. K. et al.** *, Proc. Natl. Acad.,* 2011, vol. 108, 13999-14004
- **EASTBURN, D. J. et al.** *, PloS ONE,* 2013, vol. 8, e62961
- **DALGLEISH.** *Trends in Food Science & Technology,* 1997, vol. 8 (1), 1-6
- **SCHUTZE, T. et al.** *Anal.Biochem.,* 2011, vol. 410, 155-157
- **DIEHL, F. et al.** *Nature Methods,* 2006, vol. 3, 551-559
- **GRÉGOIRE et al.** *European Journal of Immunology,* 1996, vol. 26 (10), 2410-16
- **YU, X. et al.** *J. Immunol. Methods,* 2008, vol. 336, 142-151
- **LI, Y et al.** *Nat. Biotechnol.,* 2005, vol. 23, 349-354
- **SMITH, S. N. et al.** *Methods Mol. Biol. Clifton NJ,* 2015, vol. 1319, 95-141
- **CROWE, J. E. ; KOFF, W. C.** *Expert Rev. Vaccines,* 2015, vol. 14, 1421-1425
- **LEFRANC et al.** *Nucleic Acids Research,* 2009, vol. 37
- **LLOYD, C. et al.** *Protein Eng. Des. Sel. PEDS,* 2009, vol. 22, 159-168
- **CHIU, C.-H. ; CHAO, A.** *PeerJ,* 2016, vol. 4, e1634
- **VAUGHAN, T. J. et al.** *Nat. Biotechnol.,* 1996, vol. 14, 309-314
- **BENJAMIN, E. et al.** *J. Virol.,* 2014, vol. 88, 6743-6750
- **PAPPAS, L. et al.** *Nature,* 2014, vol. 516, 418-422
- **XIAO, X. et al.** *PLoS ONE,* 2015, vol. 10, e0140691
- **DREYFUS, C. et al.** *Science,* 2012, vol. 337, 1343-1348
- **MACAGNO, A. et al.** *J. Virol.,* 2010, vol. 84, 1005-1013

64